# EUROPEAN PATENT SPECIFICATION

(11) **EP 1 334 174 B1**
(45) Date of publication and mention of the grant of the patent: **12.04.2006**
(21) Application number: 01981031.6
(22) Date of filing: 08.11.2001
(51) Int. Cl.: C12N 1/00

(54) **PARAMYXOVIRUS VECTOR FOR GENE TRANSFER TO THE CARDIOVASCULAR SYSTEM**
PARAMYXOVIRUS-VEKTOR ZUM GENTRANSFER INS KARDIOVASKULÄRE SYSTEM
VECTEUR DE PARAMYXOVIRUS DESTINE AU TRANSFERT DE GENES DANS LE SYSTEME CARDIOVASCULAIRE

(30) Priority: 08.11.2000 JP 2000339942
(43) Date of publication of application: 13.08.2003
(60) Divisional application: 06002264.7
(73) Proprietor: Dnavec Research Inc., Tsukuba-shi, Ibaraki 305-0856 (JP)
(72) Inventor: GRIESENBACH, Uta, Imp. College School of Medicine, London SW3 6LR (GB); FERRARI, Stefano, Imp. College School of Medicine, London SW3 &LR (GB); GEDDES, Duncan M., Imp. College School of Medicine, London SW3 6LR (GB); ALTON, Eric WFW, Imp. College School of Medicine, London SW3 6LR (GB); HASEGAWA, Mamoru, DNAVEC Research Inc., Tukuba-shi, Ibaraki 305-0856 (JP); HOU, Xiaogang, Alhambra, CA 91801 (US)
(74) Representative: Vossius & Partner
(86) International application number: PCT/JP2001/009786
(87) International publication number: WO 2002/038726

(56) References cited:
- EP-A- 1 013 667
- WO-A-98/51807
- YONEMITSU Y ET AL: "Efficient gene transfer to airway epithelium using recombinant Sendai virus" NATURE BIOTECHNOLOGY, NATURE PUBLISHING, US, vol. 18, no. 9, September 2000 (2000-09), pages 970-973, XP002165781 ISSN: 1087-0156
- HAI-OU LI ET AL: "A CYTOPLASMIC RNA VECTOR DERIVED FROM NONTRANSMISSIBLE SENDAI VIRUSWITH EFFICIENT GENE TRANSFER AND EXPRESSION" JOURNAL OF VIROLOGY, THE AMERICAN SOCIETY FOR MICROBIOLOGY, US, vol. 74, no. 14, July 2000 (2000-07), pages 6564-6569, XP002931595 ISSN: 0022-538X
- MORIYA C ET AL: "LARGE QUANTITY PRODUCTION WITH EXTREME CONVENIENCE OF HUMAN SDF-1ALPHA AND SDF-1BETA BY A SENDAI VIRUS VECTOR" FEBS LETTERS, ELSEVIER SCIENCE PUBLISHERS, AMSTERDAM, NL, vol. 425, no. 425, 1998, pages 105-111, XP000887284 ISSN: 0014-5793
- GRIESENBACH U ET AL: "Sendai virus-mediated overexpression of interleukin 10 following pulmonary and intramuscular administration." THORAX, vol. 55, no. Supplement 3, December 2000 (2000-12), page A5 XP008007620 Winter Meeting of the British Thoracic Society;Westminster, London, UK; December 13-15, 2000 ISSN: 0040-6376
- SHIOTANI A ET AL: "Skeletal muscle regeneration after insulin-like growth factor I gene transfer by recombinant Sendai virus vector." GENE THERAPY, vol. 8, no. 14, July 2001 (2001-07), pages 1043-1050, XP008007613 ISSN: 0969-7128
- SAKAI,Y. ET AL.: "Accomodation of foreing genes into the Sendai virus genome: sizes of inserted genes and viral replication." FEBS LETTERS, vol. 456, no. 2, 6081, pages 221-226,

## Description

### Technical Field

The present invention relates to the use of a paramyxovirus vector comprising a foreign gene encoding a secretory protein for the preparation of a pharmaceutical composition for the treatment of an inflammatory disease wherein said paramoxyvirus vector is to be administered intramuscularly.

### Background Art

Recently, gene therapy is being studied in a variety of diseases. This is a method of treatment in which a gene having a therapeutic effect is exogenously introduced into patients and expressed *in vivo.* Examples of vectors used in gene therapy are, for instance, DNA itself, DNA contained in liposomes, or virus vectors. These vectors can be either administered directly (*in vivo* gene therapy), or used to transform cells that are subsequently introduced into patients (*ex vivo* gene therapy).

For instance, diseases that may be treated by gene therapy include pulmonary fibroses such as cystic fibrosis (CF). CF is an autosomal recessive hereditary disease that causes congenital metabolic disorders. CF frequently occurs among Caucasians at a frequency of one per 2,000 to 2,500 children. In CF patients, mucous secreta accumulate in many tissues including the lungs, respiratory tract, pancreas, liver, and small intestine due to exocrine abnormalities. Control of lung infections by antibiotics and lung transplantations constitutes the main treatment in these CF patients for whom lung infections are especially fatal. In the lungs of CF patients, respiratory tract tissues are progressively destroyed by chronic inflammation. In such tissues, the balance between pro-inflammatory cytokine and anti-inflammatory cytokine production is assumed to have collapsed.

An effective way to perform gene therapy in the above case, is to administer a vector to the site of disease and locally express the therapeutic gene. It is also possible to transfer the gene product to the whole body through the cardiovascular system. In particular, when it is difficult to locally administer the vector for gene therapy, or if there is a chance of causing undesirable side effects, it may be an effective treatment strategy to express the therapeutic gene in the whole cardiovascular system, especially in the case of genes encoding biologically active substances having a short half life in blood (such as cytokines). In this context, muscles are suggested to be suitable "factories" for producing secretory proteins that are expected to be transferred through the bloodstream and function at a remote site. So far, studies of gene transfection into muscles have been using mostly naked DNA and adeno associated viruses (AAV). These vectors, however, do not achieve a sufficient expression level. Thus, it is desirable to develop a new vector that enables the secretion of a transfected gene product at a high level in the cardiovascular system.

### Disclosure of the Invention

An objective of the present invention is to provide a paramyxovirus vector for gene transfer to the cardiovascular system and uses thereof.

The endai virus, belonging to the *Paramyxoviridae* family, has recently been utilized to develop vectors for gene transfer (Kato A. et al., EMBOJ., 1997, 16, 578-598; WO97/16538, and WO97/16539). The Sendai virus vector has a low toxicity, and the amount of protein expressed from the transfected gene is extremely high. In addition, it excels in safety since the gene within the vector is not integrated into the host genome. The present inventors thought that the recombinant Sendai virus (SeV) might be utilized to efficiently express transfected gene products in the cardiovascular system. The inventors constructed a novel Sendai virus vector for expressing anti-inflammatory cytokine interleukin-10 (IL-10) (SeV-IL10), and conducted studies using it.

When transfected to COS7 cells *in vitro*, SeV-IL10 dose-dependently increased the secretion of IL-10 at 16 hr after transfection. The secretion level of IL-10 with the highest titer of SeV-IL10 (10⁶ pfu/well in 24-well plate) was higher by two orders of magnitude than that achieved by liposome-mediated transfection of a plasmid encoding IL-10. Therefore, the inventors performed *in vivo* transfection experiments using the SeV-IL10 vector.

SeV-IL10 was locally administered into the mouse respiratory tract via intranasal drops. Two days later, the level of IL-10 secreted into the lung homogenate and broncho-alveolar lavage fluid (BALF) was measured, and compared with that obtained by plasmid DNA transfection mediated by liposomes. The level of IL-10 secretion obtained by SeV-IL10 administration was higher by two orders of magnitude in the lung homogenate than that obtained by plasmid transfection (SeV-IL10: 21457±5112 pg/mg protein; plasmid: 310±54 pg/mg protein), and it was higher by three orders of magnitude in BALF (SeV-IL10: 153366±41823 pg/ml; plasmid: 71±63 pg/ml). The IL-10 level in blood was further examined. In the mouse given SeV-IL10, a significant amount of IL-10 was secreted.

Furthermore, SeV-IL10 was injected into a skeletal muscle, which is a suitable site for production of secretory proteins, and the effect was examined. SeV-IL10 vector (4 x 10⁸ pfu/muscle) was injected into the tibialis anterior muscle and the IL-10 expression level was examined two days later. The expression level in the muscle homogenate was higher by one to two orders of magnitude than that in the case of plasmid DNA injection (50 µg DNA) (SeV-IL10: 1067±32 pg/mg protein; plasmid: 50.9±11 pg/mg protein). IL-10 was not detected in the serum by plasmid injection, whereas a significant increase in serum IL-10 level was achieved two days after injection of SeV-IL10 vector (SeV-IL10: 393±132 pg/ml; SeV-βgal: 0.31±0.26 pg/ml). Taken together, the efficiency of gene transfer into lungs and muscles using the recombinant SeV was very high, suggesting that SeV vector can express products of transfected genes at high levels in the cardiovascular system. The vector disclosed herein is useful as a vector for gene therapy against a variety of diseases that are curable by transferring genes into the cardiovascular system. Especially, the vector enables the application of gene therapy to pneumonia in CF patients.

The present invention enables the efficient transfer of genes by the intramuscular administration of recombinant SeV. The expression level of a transfected gene reaches a high level two days after injection. This level is significantly higher than that obtained using plasmid DNA or AAV. The SeV-mediated production of therapeutic gene products is extremely effective under clinical conditions, where a high level of gene expression is required quickly. When it is difficult to rule out the possibility that the vector itself may have an inflammatory effect, it is advantageous to administer vectors into a muscle far from the site of disease to avoid worsening inflammation, compared to direct administration to the site. Accordingly, intramuscular administration of the vector of the invention is expected to enable a more effective treatment. Moreover, the use of SeV lacking a replication ability would reduce inflammatory reactions and antibody reactions observed when using vectors that can replicate.

Thus, the present invention relates to the use of a paramyxovirus vector for gene transfer to the cardiovascular system as specified below:
(1) use of a paramyxovirus vector comprising a foreign gene encoding a secretory protein for the preparation of a pharmaceutical composition for the treatment of an inflammatory disease, wherein said paramyxovirus vector is to be administered intramuscularly;
(2) the use according to (1), wherein said secretory protein is an anti-inflammatory cytokine;
(3) the use according to (2), wherein said anti-inflammatory cytokine is IL-10;;
(4) the use according to (1), (2) or 3, wherein said paramyxovirus is sendai virus; and
(5) the use according to (1), wherein said inflammatory disease is pulmonary fibrosis.

Herein, a "paramyxovirus vector" is defined as a vector (or carrier) that is derived from the paramyxovirus and that is used for gene transfer to host cells. The paramyxovirus vector disclosed herein may be ribonucleoprotein (RNP) or a virus particle having infectivity. Here, "infectivity" is defined as the ability of the recombinant paramyxovirus vector to transfer, through its cell adhesion and membrane fusion abilities, a gene contained in the vector to cells to which the vector is adhered. The paramyxovirus vector disclosed herein carries a foreign gene in an expressible manner. The paramyxovirus vector may have a replication ability, or may be a defective vector without the replication ability. Herein, "replication ability" is defined as the ability of virus vectors to replicate and produce infective virus particles in host cells infected with the virus vectors.

Herein, a "recombinant" paramyxovirus vector is defined as one constructed by gene engineering or its amplified products. For instance, recombinant paramyxovirus vectors can be generated by reconstitution of a recombinant paramyxovirus cDNA.

Herein, a paramyxovirus is defined as a virus of the *Paramyxoviridae* family or a derivative thereof. The present invention can be applied to, for example, paramyxoviruses such as Sendai virus, Newcastle disease virus, Mumps virus, Measles virus, Respiratory syncytial virus, rinderpest virus, distemper virus, simian parainfluenza virus (SV5), type I, II, and III human parainfluenza virus of the *Paramyxoviridae*. The virus disclosed herein may be preferably a virus of the genus *Paramyxovirus* or a derivative thereof. Viruses of the genus *Paramyxovirus* to which the present invention is applicable include human parainfluenza virus type 1 (HPIV-1), human parainfluenza virus type 3 (HPIV-3), bovine parainfluenza virus type 3 (BPIV-3), Sendai virus (also called mouse parainfluenza virus type 1), simian parainfluenza virus type 10 (SPIV-10), and many other viruses of the genus *Paramyxovirus*. The paramyxovirus is most preferably Sendai virus. These viruses may be wild-type strains, mutant strains, laboratory-passaged strains, artificially constructed strains, or so on. Incomplete viruses such as the DI particle (Willenbrink W. and Neubert W. J., J. Virol., 1994, 68, 8413-8417), synthesized oligonucleotides, and so on, may also be utilized as material for generating the virus vector disclosed herein.

Genes encoding proteins of a paramyxovirus include NP, P, M, F, HN, and L genes. Here, the "NP, P, M, F, HN, and' L genes" represent those encoding the nucleocapsid protein, phosphoprotein, matrix protein, fusion protein, hemagglutinin-neuraminidase, and large protein, respectively. Genes of each virus of the subfamily paramyxovirus are described generally as follows. In general, NP gene may also be indicated as "N gene".

| | | | | | | |
|---|---|---|---|---|---|---|
| *Paramyxovirus* | NP | P/C/VM | F | HN | - | L |
| *Rublavirus* | NP | P/V M | F | HN | (SH) | L |
| *Morbillivirus* | NP | P/C/VM | F | H | - | L |

For instance, the accession numbers in the nucleotide sequence database of each gene of the Sendai virus classified as a Respirovirus of *Paramyxoviridae*, are M29343 M30202, M30203, M30204, M51331, M55565, M69046, and X17218 for the NP gene; M30202, M30203, M30204, M55565, M69046, X00583, X17007, and X17008 for the P gene; D11446, K02742, M30202, M30203, M30204, M69046, U31956, X00584, X53056 for the M gene; D00152, D11446, D17334 D17335, M30202, M30203, M30204, M69046, X00152, and X02131 for the F gene; D26475, M12397, M30202, M30203, M30204, M69046, X00586, X02808, X56131 for the HN gene; and D00053, M30202, M30203, M30204, M69040, X00587, and X58886 for the L gene.

Here, a "gene" is defined as a genetic substance, which includes nucleic acids such as RNA and DNA. In general, a gene may or may not encode a protein. For example, a gene may be a gene encoding a functional RNA such as ribozyme, antisense RNA, etc. Genes may have naturally derived or artificially designed sequences. Herein, "gene transfer" is defined as gene-mediated transfer, and "gene transfer to the cardiovascular system" includes transferring the products of genes to the system. A "secretory protein" is defined as a protein secreted to the outside of cells. The protein may not necessarily have an obvious secretion signal as long as it can be secreted to the outside. The secretory protein may be a naturally derived or artificially designed protein. It is possible to secrete a desired protein to the outside by adding a secretion signal. An artificially designedprotein, for instance, maybe a fusion protein with another protein, a dominant negative protein, including a soluble form of a receptor or a membrane-bound dominant negative receptor, a deletion form of a cell adhesion molecule, and a soluble form of a cell surface molecule. Herein, "DNA" includes single stranded DNA or a double stranded DNA.

Herein, cytokines are defined as all proteins and polypeptides other than antibodies, which are secreted from cells and exhibit physiological functions such as the regulation of the immune system, an antitumor function, an antivirus function, or the regulation of cell differentiation (Aggarwal B.B. and Pocsik E., "Cytokines: from clone to clinic," Arch. Biochem. Biophys., 1992, 292(2), 335-359). Lymphokines and monokines are substantially the same as cytokines, and are included in the cytokine of the present invention. In addition, anti-inflammatory cytokines are def ined as cytokines that inhibit synthesis of one ormore cytokines that are secreted from any of Th1 cells, NK cells, monocytes, and macrophages (such as INF-γ, TNF-β, IL-2, IL-1, IL-6, IL-8, or TNF-α) Cytokines may be natural proteins or artificially altered proteins. Anti-inflammatory cytokines include IL-10, IL-4, and IL-12, but are not limited thereto. Genes encoding those cytokines can be prepared by a known method such as PCR using primers that are designed based on their nucleotide sequence. Most preferably, the anti-inflammatory cytokine of the present invention is IL-10.

Disclosed herein is the use of a paramyxovirus vector for gene transfer to the cardiovascular system. The present inventors have succeeded in overexpressing a product of transfected gene not only at the site of injection but also in blood by *in vivo* administration of a paramyxovirus vector encoding a foreign gene. Sendai virus vector for expression of IL-10 (SeV-IL10) intranasally administered to mice by sniffing was introduced into the nasal epithelia (turbinate) and lung. The gene product was overexpressed at the site of injection and a significant increase in IL-10 level was also observed in blood. Administration by perfusion into the nasal epithelia (turbinate) also resulted in a significant increase of IL-10 level in blood. Furthermore, administration of SeV-IL10 into a skeletal muscle, which is supposed to be a suitable site for producing secretory proteins, was also examined. When SeV-IL10 was administered to the tibialis anterior (TA) muscle, the level of IL-10 expression was significantly increased not only in the muscle that is the site of injection, but also in blood. In addition, intramuscular administration of SeV-IL10 significantly inhibited collagen deposition in the lung of pulmonary fibrosis model mice prepared by bleomycin administration. Thus, the therapeutic effect of intramuscular injection of SeV-IL10 on pulmonary fibrosis was confirmed. The vector disclosed herein is extremely useful for the transfer of therapeutic gene products to the whole cardiovascular system. For instance, it is possible to perform gene therapy for various inflammatory diseases by expressing an anti-inflammatory cytokine using the vector of the present invention. It is also possible to utilize the vector for gene therapy using a gene having an anti-inflammatory function to reduce the inflammation and inhibit the accumulation of mucous secretions.

The present inventors showed that the genes introduced by intramuscular administration of recombinant paramyxovirus vectors were expressed at the peak level two days after injection and that they were persistently expressed over a week. In addition, repetitive administration increased the gene expression. These features are advantageous in obtaining a fast and sustained therapeutic effectin gene therapy using recombinant paramyxovirus vectors.

Paramyxovirus vectors can be preferably utilized in clinical trials of human gene therapy in terms of safety as well. First, it is a major obstacle in high efficient gene transfer that transfected DNA must be transported into the nucleus for the expression of a foreign gene. In the case of Sendai virus and such, however, expression of a foreign gene is driven by both cellular tubulin and its RNA polymerase (L protein) in the cytoplasm. This suggests that the Sendai virus does not interact with the genome of host cells, which avoids safety problems such as tumorigenesis. Second, the Sendai virus is known to be pathogenic in rodents causing pneumonia, but not in humans, which is supported by studies showing that the intranasal administration of the wild type Sendai virus does not do harm in nonhuman primates (Hurwitz J. L. et al., Vaccine, 1997, 15, 533-540). These features suggest that Sendai virus vector can be utilized in human therapy, and further, support the notion that Sendai virus can be one of the promising alternatives in gene therapy that is aimed at transferring products of a foreign gene to the cardiovascular system.

The vector used according to the present invention may be preferably utilized in gene therapy using anti-inflammatory cytokines, specifically targeting inflammatory diseases. The paramyxovirus vector for expressing an anti-inflammatory cytokine is useful especially for the treatment of inflammatory diseases. In other words, the introduction of genes encoding an anti-inflammatory cytokine by use of the paramyxovirus vector can inhibit inflammation and relieve disease symptoms. The diseases include, for example, pulmonary fibrosis, sclerosing peritonitis, prostatomegaly, multiple sclerosis, post transplant rejection, type I diabetes, chronic articular rheumatism, inflammatory enteropathy, psoriasis, systemic lupus erythematosus, iritis, granulomatous diseases, chronic nephritis, scleroderma, hysteromyoma, keloid, cirrhosis, and other diseases accompanied by inflammation. Herein, treatment of diseases includes the therapy and prevention of diseases.

The paramyxovirus vector used for gene transfer to the cardiovascular system is not limited to any special kind. For instance, vectors that have the replication ability and that are capable of autonomous propagation may be preferably utilized. In general, the genome of the wild type paramyxovirus contains a short 3' leader region followed by six genes encoding N (nucleocapsid), P (phospho), M (matrix), F (fusion), HN (hemagglutinin-neuraminidase), and L (large) proteins, and has a short 5' trailer region on the other terminus. The vector that is able to replicate autonomously can be obtained by designing a genome having a similar structure to that described above. In addition, a vector for expressing a foreign gene can be obtained by inserting the foreign gene to the genome of the above vector. The paramyxovirus vector may have an altered alignment of virus genes, compared with wild type virus.

The paramyxovirus vector may have deletion(s) of some of the genes that are contained in the wild type virus. For instance, in the case of the reconstitution of the Sendai virus vector, proteins encoded by NP, P/C, and L genes are thought to be required in *trans,* but the genes may not be a component of the virus vector. An expression vector carrying genes encoding the proteins may be co-transfected into host cells with another expression vector encoding the vector genome to reconstitute a virus vector. Alternatively, an expression vector encoding the virus genome is transfected into host cells carrying genes encoding the proteins, and thus a virus vector can be reconstituted by using the proteins provided by the host cell. The amino acid sequence of these proteins may not be identical to those derived from the original virus as long as it has an equivalent or higher activity in nucleic acid transfer, and may be mutated or replaced with that of a homologous gene of another virus.

Proteins encoded by M, F, and HN genes are thought to be essential for cell-to-cell propagation of a paramyxovirus vector. However, these proteins are not required when the vector is prepared as RNP. If genes M, F, and HN are components of the genome contained in RNP, products of these genes are produced when introduced into host cells, and virus particles having infectivity are generated. RNP vectors that produce an infective virus include an RNP that contains a virus genome RNA encoding N, P, M, F, HN, and L genes and N, P, and L proteins. When such RNP is introduced into cells, virus genome is expressed and replicated through functions of the proteins N, P, and L, and thus infective virus vectors are amplified.

RNP can be introduced into cells as a complex formed with lipofectamine, polycationicliposome, and the like. Specifically, a variety of transfection reagents can be used, for instance, DOTMA (Boehringer), Superfect (QIAGEN #301305), DOTAP, DOPE, DOSPER (Boehringer #1811169). Chloroquine may be added to prevent degradation in the endosome (Calos M. P., Proc. Natl. Acad. Sci. USA, 1983, 80, 3015). In the case of replicative viruses, the produced viruses can be amplified or passaged by re-infection into cultured cells, chicken eggs, or animals (e.g. mammalian such as mice).

In contrast, the paramyxovirus vector may be lacking the M, F, and/or HN genes. These vectors can be reconstituted by providing deleted gene products exogenously. Such vectors can still adhere to host cells and induce cell fusion as the wild type. However, daughter virus particles that have the same infectivity as the original ones are not produced because the vector genome introduced into cells lacks one of the above genes. Therefore, these vectors can be useful as safe virus vectors that are capable of only a single gene transfer. For instance, genes deleted from the genome may be F and/or HN genes. Virus vectors can be reconstituted by co-transfection of an expression plasmid encoding the genome of a recombinant paramyxovirus lacking the F gene, an expression vector for the F protein, and that for NP, P/C, and L proteins into host cells (WO00/70055 and WO00/70070). Alternatively, host cells in which the F gene is integrated into the chromosome may be used. The amino acid sequence of these proteins provided exogenously may not be identical to those of the wild type and may be mutated or replaced by a homologous protein of another virus as long as they provide equivalent or higher gene transfer activity.

The envelope protein of the paramyxovirus vector may contain another protein than the envelope protein of the original vector genome. There is no limitation on such proteins. These may include envelope proteins of other viruses such as the G protein of the vesicular stomatitis virus (VSV-G). Thus, the paramyxovirus vector includes a pseudo type virus vector that has an envelope protein derived from a virus different from the original virus.

Also, the paramyxovirus vector may have on the surf ace of its envelope a protein targeted at particular cells such as adhesion molecules, ligands, and receptors, or a chimeric protein having these proteins in its extracellular domain and a polypeptide derived from the virus envelope protein in its intracellular domain. It enables the production of a vector targeting a particular tissue. These proteins may be encoded by the virus genome itself, or supplied at the time of virus reconstitution through expression of genes other than virus genome (for example, another expression vector or host cell chromosome).

The virus genes contained in the vector of the invention may be altered to reduce antigenicity or enhance RNA transcription efficiency or replication efficiency. Specifically, it is possible to alter at least one of the NP, P/C, and L genes,' which are genes of replication factors, to enhance transcription or replication. It is also possible to alter the HN protein, a structural protein having hemagglutinin activity and neuraminidase activity, to enhance the virus stability in blood by weakening the former activity and to regulate infectivity by altering the latter activity. It is also possible to alter the F protein, which is implicated in membrane fusion, to regulate the fusion ability. Furthermore, it is possible to generate a paramyxovirus vector that is engineered to have weak antigenicity through analyzing the antigen presenting epitopes and such of possible antigenic molecules on the cell surface such as the F protein and HN protein.

In addition, a paramyxovirus whose accessory gene is deficient can be used in accordance with the present invention. For example, by knocking out the V gene, one of the accessory genes of SeV, pathogenicity of SeV to hosts such as mice markedly decreases without damages to the expression and replication of genes in cultured cells (Kato, A. et al., J. Virol., 1997, 71, 7266-7272; Kato, A. et al., EMBO J., 1997, 16, 578-587: Curran, J. et al., WO01/04272, EP1067179). Such attenuated vectors are particularly preferable as virus vectors for in vivo or ex vivo gene transfer.

The virus vector disclosed herein may contain a foreign gene in the genome RNA. A recombinant paramyxovirus vector containing a foreign gene can be obtained by inserting the gene into the genome of the above-described paramyxovirus vector. The foreign gene may be a gene encoding a desired protein to be expressed in blood. It may encode a natural protein, or an altered protein having a deletion, substitution, or insertion as long as the protein has a function equivalent to that of the natural protein. Alternatively, it may be an artificially designed protein such as a dominant negative mutant. For instance, for the purpose of gene therapy and the like, a gene used to treat a target disease may be inserted into the DNA encoding the genome of the virus vector (the virus vector DNA). In the case of inserting a foreign gene into Sendai virus vector DNA, a sequence comprising nucleotides of multiples of six is desirably inserted between the transcription end sequence (E) and the transcription start sequence (S) (Calain P. and Roux L., J. Virol., 1993, 67(8), 4822-4830). A foreign gene can be inserted upstream and/or downstream of each of the virus genes (NP, P, M, F, HN, and L genes). In order not to interfere with the expression of upstream and downstream genes, an E-I-S sequence (transcription end sequence-intervening sequence-transcription start sequence) or a portion of it may be suitably placed upstream or downstream of a foreign gene so that the E-I-S sequence is located between each gene. Alternatively, a foreign gene can be inserted with IRES.

The expression level of inserted genes can be regulated by the type of transcription start sequence that is attached to the upstream of the genes (WO01/18223). It also can be regulated by the position of insertion and the sequence surrounding the gene. In the Sendai virus, for instance, the closer to the 3'-terminus of the negative strand RNA of the virus genome (the closer to NP gene in the gene arrangement on the wild type virus genome) the insertion position is, the higher the expression level of the inserted gene will be. To achieve a high expression of a foreign gene, it is preferably inserted into the upstream region of the negative stranded genome such as the upstream of the NP gene (3' flanking region on the negative strand), or between NP and P genes. Conversely, the closer to the 5'-terminus of the negative strand RNA (the closer to L gene in the gene arrangement on the wild type virus genome) the insertion position is, the lower the expression level of the inserted gene will be. To reduce the expression of a foreign gene, it may be inserted into the most 5' position on the negative strand, that is, downstream of the L gene in the wild type virus genome (5' flanking region of the L gene on the negative strand) or upstream of the L gene (3' flanking region of L gene on the negative strand). Thus, the insertion position of a foreign gene can be properly adjusted so as to obtain a desired expression level of the gene or to optimize the combination of the insert with the virus genes surrounding it. For instance, if the overexpression of a gene introduced by a high titer virus vector may cause toxicity, it is possible not only to control the virus titer, but also to reduce the expression level of individual vectors by designing the insertion position closer to the 5'-terminus of the negative strand, or replacing the transcription start sequence with one having lower efficiency so as to obtain an appropriate therapeutic effect.

To help the easy insertion of a foreign gene, a cloning site may be designed at the position of insertion. For example, the cloning site may be the recognition sequence of restriction enzymes. The restriction sites in the virus vector DNA can be used to insert a foreign gene. The cloning site may be a multicloning site that contains recognition sequences for multiple restriction enzymes. The vector of the present invention may have other foreign genes at positions other than that used for the above insertion. Such foreign genes are not limited but may be other anti-inflammatory cytokine genes, or may be other kinds of genes.

Construction of a recombinant Sendai virus vector having a foreign gene can be performed as follows, for example, according to the method described (Hasan M. K. et al., J. Gen. Virol., 1997, 78, 2813-2820; Kato A. et al., EMBO J., 1997, 16, 578-587; Yu D. et al., Genes Cells, 1997, 2, 457-466).

First, a DNA sample comprising the cDNA nucleotide sequence of a desired foreign gene is prepared. It is preferable that the DNA sample can be electrophoretically identified as a single plasmid at concentrations of 25 ng/µl or more. Below, a case where a foreign gene is inserted into DNA encoding the viral genome utilizing a NotI site will be described as an example. When the NotI recognition site is included in the objective cDNA nucleotide sequence, it is preferable to delete the NotI site beforehand by modifying the nucleotide sequence using site-specific mutagenesis and such method so as not to alter the amino acid sequence encoded by the cDNA. From this DNA sample, the desired gene fragment is amplified and recovered by PCR. To have NotI sites on both ends of the amplified DNA fragment and to further add a copy of transcription termination sequence (E), intervening sequence (I) and transcription initiation sequence (S) (EIS sequence) of Sendai virus to one end, a forward side synthetic DNA sequence (sense strand) and reverse side synthetic DNA sequence (antisense strand) are prepared as a pair of primers containing NotI restriction enzyme cleavage site sequence, transcription termination sequence (E), intervening sequence (I), transcription initiation sequence (S) and a partial sequence of the objective gene.

For example, to secure cleavage by NotI, the forward side synthetic DNA sequence is arranged in a form in which any two or more nucleotides (preferably 4 nucleotides excluding GCG and GCC, sequences originating in NotI recognition site, more preferably ACTT) are selected on the 5'-side of the synthetic DNA, NotI recognition site "gcggccgc" is added to its 3'-side, and to the 3'-side thereof, any desired 9 nucleotides or nucleotides of 9 plus a multiple of 6 nucleotides are added as the spacer sequence, and to the 3'-side thereof, about 25 nucleotide-equivalent ORF including the initiation codon ATG of the desired cDNA is added. It is preferable to select about 25 nucleotides from the desired cDNA as the forward side synthetic DNA sequence so as to have G or C as the final nucleotide on its 3'-end.

In the reverse side synthetic DNA sequence, any two or more nucleotides (preferably 4 nucleotides excluding GCG and GCC, sequences originating in the NotI recognition site, more preferably ACTT) are selected on the 5'-side of the synthetic DNA, NotI recognition site "gcggccgc" is added to its 3'-side, and to its further 3'-side, an oligo DNA is added as the insertion fragment to adjust the length. This oligo DNA is designed so that the total nucleotide number including the NotI recognition site "gcggccgc", complementary sequence of cDNA and EIS nucleotide sequence of Sendai virus genome originating in the virus described below becomes a multiple of six (so-called "rule of six"; Kolakofski D. et al., J. Virol., 1998, 72, 891-899; Calain P. and Roux L., J. Virol., 1993, 67, 4822-4830; Calain, P. and Roux, L., J. Virol., 1993, 67, 4822-4830). Further to the 3'-side of inserted fragment, a sequence complementary to the S sequence of the Sendai virus, preferably t 5' -CTTTCACCCT-3' (SEQ ID NO: 1), a sequence complementary to the I sequence, preferably 5'-AAG-3' , and a sequence complementary to the E sequence, preferably 5'-TTTTTCTTACTACGG-3' (SEQ ID NO: 2), is added, and further to the 3' -side thereof, an about 25 nucleotide-equivalent complementary sequence counted in the reverse direction from the termination codon of the desired cDNA sequence the length of which is adjusted to have G or C as the final nucleotide, is selected and added as the 3'-end of the reverse side synthetic DNA.

PCR can be done according to the usual method with, for example, ExTaq polymerase (Takara Shuzo). Preferably, PCR is performed using Vent polymerase (NEB), and desired fragments thus amplified are digested with NotI, then inserted to NotI site of the plasmid vector pBluescript. Nucleotide sequences of PCR products thus obtained are confirmed with a sequencer to select a plasmid having the right sequence. The inserted fragment is excised from the plasmid using NotI, and cloned to the NotI site of the plasmid carrying the genomic cDNA. Alternatively, it is also possible to obtain the recombinant Sendai virus cDNA by directly inserting the fragment to the NotI site without the mediation of the plasmid vector pBluescript.

For example, recombinant Sendai virus genomic cDNA can be constructed according to the methods in the literature (Kato A. et al., EMBO J., 1997, 16, 578-598; Hasan M. K. et al., J. Gen. Virol., 1997, 78, 2813-2820; Yu, D. et al., Genes Cells, 1997, 2, 457-466). For instance, a spacer sequence of 18 bp containing the NotI site (5'-(G)-CGGCCGCAGATCTTCACG-3'; SEQ ID NO: 3) is inserted into an adjacent gene locus of a cloned Sendai virus genomic cDNA (pSeV(+)) between the leader sequence and the 5'-terminus of a sequence encoding the N protein, and the plasmid pSeV18⁺b (+) containing a self-cleavable ribozyme site derived from the antigenomic strand of the delta hepatitis virus is obtained (Hasan M. K. et al., J. General Virol., 1997, 78, 2813-2820). A foreign gene fragment is inserted into the NotI site of pSeV18⁺b(+) to obtain a recombinant Sendai virus cDNA into which a desired foreign gene has been inserted.

The recombinant paramyxovirus vector DNA is transcribed in vitro or in cells, and RNP is reconstituted in the presence of L, P, and NP proteins to generate a virus vector comprising the RNP. A method for producing the paramyxovirus vector may comprise transcribing a DNA encoding the genome of the virus vector. A DNA for producing the paramyxovirus vector, may comprise the DNA encoding the genome of the virus vector. The use of a DNA encoding the genome of the vector to produce the paramyxovirus vector of the invention is also disclosed herein. Reconstitution of a virus from virus vector DNA can be performed according to the known methods (WO97/16539 WO97/16538 Durbin A. P. et al., Virol., 1997, 235, 323-332; Whelan S. P. et al., Proc. Natl. Acad. Sci. USA, 1995, 92, 8388-8392; Schnell M. J. et al., EMBO J. , 1994, 13, 4195-4203; Radecke F. et al., EMBO J., 1995, 14, 5773-5784; Lawson N. D. et al., Proc. Natl. Acad. Sci. USA, 1995, 92, 4477-4481; Garcin D. et al., EMBO J., 1995, 14, 6087-6094; Kato A. et al., Genes Cells, 1996, 1, 569-579; Baron M. D. and Barrett T., J. Virol., 1997, 71, 1265-1271; Bridgen A. and Elliott R. M., Proc. Natl. Acad. Sci. USA, 1996, 93, 15400-15404). These methods enable reconstituting, from DNA, desired paramyxovirus vectors including the parainfluenza virus, vesicular stomatitis virus, rabies virus, measles virus, rinderpest virus, Sendai virus vectors, etc. When a viral vector DNA is made deficient in F, HN and/or M genes, infectious virus particles are not formed with such a defective vector. However, it is possible to form infectious virus particles by separately transferring these deficient genes, genes encoding other viral envelope proteins, and the like, to host cells and expressing them therein.

Methods for transferring viral vector DNA into cells include the following: 1) the method of preparing DNA precipitates that can be taken up by objective cells; 2) the method of preparing a positively charged complex comprising DNA, which is suitable for being taken up by objective cells and which is also not very cytotoxic, and 3) the method of instantaneously bering on the objective cellular membrane pores wide enough to allow DNA molecules to pass through by electric pulse.

In Method 2), a variety of transfection reagents can be utilized, examples being DOTMA (Boehringer), Superfect (QIAGEN #301305), DOTAP, DOPE, DOSPER (Boehringer #1811169), etc. An example of Method 1) is a transfection method using calcium phosphate, in which DNA that entered cells are incorporated into phagosomes, and a sufficient amount is incorporated into the nuclei as well (Graham, F. L. and Van Der Eb, J., Virology, 1973, 52, 456; Wigler, M. and Silverstein, S., Cell, 1977, 11, 223) . Chen and Okayama have investigated the optimization of the transfer technique, reporting that optimal DNA precipitates can be obtained under conditions where 1) cells are incubated with DNA in an atmosphere of 2 to 4% CO₂ at 35°C for 15 to 24 h, 2) cyclic DNA with a higher precipitate-forming activity than linear DNA is used, and 3) optimal DNA concentration in the precipitate mixture is 20 to 30 µg/ml (Chen, C. and Okayama, H., 1987, Mol-. Cell. Biol. 7: 2745). Method 2) is suitable for a transient transfection. An old method is known in the art in which a DEAE-dextran (Sigma #D-9885, M.W. 5 x 10⁵) mixture is prepared in a desired DNA concentration ratio to perform the transfection. Since most of the complexes are decomposed inside endosomes, chloroquine may be added to enhance transfection efficiency (Calos M. P., Proc. Natl. Acad. Sci. USA, 1983, 80, 3015). Method 3) is referred to as electroporation, and is more versatile compared to methods 1) and 2) because it doesn't have cell selectivity. Method 3) is said to be efficient under optimal conditions for pulse electric current duration, pulse shape, electric field potency (gap between electrodes, voltage), conductivity of buffers, DNA concentration, and cell density.

Among the above-described three categories, transfection reagents (method 2) are suitable in the present invention, because method 2) is easily operable, and facilitates the examining of many test samples using a large amount of cells. Preferably, Superfect Transfection Reagent (QIAGEN, Cat. No. 301305) or DOSPER Liposomal Transfection Reagent (Boehringer Mannheim, Cat. No. 1811169) is used, but the transfection reagents are not limited thereto.

Specifically, the reconstitution of the viral vector from cDNA can be performed as follows.

A simian kidney-derived cell line LLC-MK2 is cultured in 24-well to 6-well plastic culture plates or 100 mm diameter culture dishes or the like using a minimum essential medium (MEM) containing 10% fetal calf serum (FCS) and antibiotics (100 units/ml penicillin G and 100 µg/ml streptomycin) to 70 to 80% confluency, and infected, for example, at 2 PFU/cell with recombinant vaccinia virus vTF7-3 expressing T7 polymerase which has been inactivated by a UV irradiation treatment for 20 min in the presence of 1 µg/ml psoralen (Fuerst, T. R. et al., Proc. Natl. Acad. Sci. USA 83: 8122-8126, 1986; Kato, A. et al., Genes Cells 1: 569-579, 1996) . Amount of psoralen added and UV irradiation time can be appropriately adjusted. One hour after the infection, the cells are transfected with 2 to 60 µg, more preferably 3 to 5 µg, of the above-described recombinant Sendai virus cDNA by the lipofection method or the like using plasmids (24 to 0.5 µg of pGEM-N, 12 to 0.25 µg of pGEM-P and 24 to 0.5 µg of pGEM-L, more preferably 1 µg of pGEM-N, 0.5 µg of pGEM-P and 1 µg of pGEM-L) (Kato, A. et al., Genes Cells, 1996, 1, 569-579) expressing *trans*-acting viral proteins required for the production of full-length Sendai viral genome together with Superfect (QIAGEN). The transfected cells are cultured in a serum-free MEM containing 100 µg/ml each of rifampicin (Sigma) and cytosine arabinoside (AraC) if desired, more preferably only containing 40 µg/ml of cytosine arabinoside (AraC) (Sigma), and concentrations of reagents are set at optima so as to minimize cytotoxicity due to the vaccinia virus and maximize the recovery rate of the virus (Kato. A. et al., Genes Cells, 1996, 1, 569-579). After culturing for about 48 to 72 h following the transfection, the cells are recovered, disrupted by repeating three cycles of freezing and thawing, transfected to LLC-MK2 cells, and cultured. After culturing the cells for 3 to 7 days, the culture solution is collected. To reconstitute a virus vector lacking a gene encoding an envelope protein that is incapable of replication, the vector may be transfected into LLC-MK2 cells expressing an envelope protein, or co-transfected with expression plasmid for the envelope protein. Alternatively, LLC-MK2 cells expressing envelope protein can be overlaid on transfected cells and cultured to propagate a deletion virus vector (WO00/70055 and WO00/70070). Virus titer contained in the culture supernatant can be determined by measuring the hemagglutination activity (HA), which can be assayed by the "endo-point dilution method" (Kato. A. et al., Genes Cells, 1996, 1, 569-579; Yonemitsu Y. and Kaneda Y., Hemagglutinating virus of Japan-liposome-mediated gene delivery to vascular cells., Molecular Biology of Vascular Diseases. Methods in Molecular Medicine, Ed. by Baker A. H., Humana Press, 1999, 295-306). The possible contamination of vaccinia virus vTF7-3 can be eliminated by re-amplifying in chicken eggs after the obtained allantoic sample is diluted appropriately (10⁶ times for instance).
Re-amplification may be repeated, for example, three times or more. Virus stock thus obtained can be stored at -80°C.

The type of host cells used for virus reconstitution is not particularly limited, so long as viral vector can be reconstituted therein. For example, culture cells such as simian kidney-derived CV-1 cells and LLC-MK2 cells, hamster kidney-derived BHK cells, human-derived cells, and so on can be used. To obtain the Sendai virus vector in a large quantity, the vector can be amplified, for example, by infecting virus vector obtained from the above-described host cells into embryonated chicken eggs. Methods for manufacturing viral fluid using chicken eggs have already been developed (Nakanishi, et al. (eds.), 1993, "Shinkei-kagaku Kenkyu-no Sentan-gijutu Protocol III (High Technology Protocol III of Neuroscience Research), Molecular Neurocyte Physiology, Koseisha, Osaka, pp.153-172). Specifically, for example, fertilized eggs are placed in an incubator and incubated for 9 to 12 days at 37 to 38 °C to grow embryos. The Sendai virus vector is inoculated into the allantoic cavity of eggs, and cultured for several days to proliferate the virus. Conditions such as culture duration may be varied depending on the type of recombinant Sendai virus used. Subsequently, allantoic fluid comprising the virus is recovered. Separation and purification of the Sendai virus vector can be performed according to the standard methods (Tashiro, M., "Virus Experiment Protocols", Nagai and Ishihama (eds.), Medicalview, 1995, 68-73).

For instance, a Sendai virus vector lacking the F protein can be constructed and prepared as follows (WO00/70055 and WO00/70070).

### (1) Construction of Sendai virus genome cDNA lacking the F gene and an expression plasmid for F gene

Full length Sendai virus (SeV) genomic cDNA, pSeV18⁺b(+) (Hasan M. K. et al., J. General Virol., 1997, 78, 2813-2820) (pSeV18⁺b(+) may be also called pSeV18⁺), is digested with SphI and KpnI, and the resulting fragment (14673 bp) is recovered and cloned into pUC18 to obtain pUC18/KS- pUC18/KS is used for constructing a region lacking the F gene. Deletion of the F gene is performed by combination of PCR-ligation, and the ORF of the F gene (1698 bp, from ATG to TGA) is replaced with the sequence 5'-atgcatgccggcagatga (SEQ ID NO: 4) in the resulting F gene-deleted SeVgenomiccDNA (pSeV18⁺/ΔF). PCRproducts obtained using primers (forward: 5'-gttgagtactgcaagagc/SEQ ID NO: 5; reverse: 5'-tttgccggcatgcatgtttcccaaggggagagttttgcaacc/SEQ ID NO: 6) and those with primers (forward: 5'-atgcatgccggcagatga/SEQ ID NO: 7; reverse: 5'-tgggtgaatgagagaatcagc/SEQ ID NO: 8) are digested with EcoT22I and cloned into the upstream and downstream of the F gene, respectively. The resulting plasmid is digested with SacI and SalI, and the fragment containing the F gene deletion site (4931 bp) is recovered and cloned into pUC18 to obtain pUC18/dFSS. pUC18/dFSS is digested with DraIII, and the fragment recovered is replaced with the DraIII fragment of pSeV18⁺ that contains F gene, and ligated to obtain pSeV18⁺/ΔF.

A foreign gene can be inserted into the NsiI or NgoMIV site in the F gene deletion site of pUC18/dFSS. For this purpose, a fragment containing a foreign gene may be amplified using NsiI-tailed primers or NgoMIV-tailed primers.

### (2) Preparation of helper cells for inducible expression of SeV-F protein

A Cre/loxP inducible expression plasmid for the Sendai virus F gene (SeV-F) is constructed as follows. SeV-F gene is amplified by PCR, and cloned into the unique SwaI site of the pCALNdLw plasmid (Arai T. et al., J. Virol., 1998, 72, 1115-1121), which is designed for inducible expression of gene products through the function of Cre DNA recombinase, to obtain pCALNdLw/F.

To recover infectious virus particles from the Fgene-deleted genome, a helper cell line expressing SeV-Fprotein is established. LLC-MK2 cells, derived from the Simian kidney and commonly used for SeV propagation, may be used. LLC-MK2 cells are cultured at 37 °C, 5% CO₂ in MEM containing 10% heat-inactivated and immobilized fetal bovine serum (FBS), 50 U/ml of penicillin G sodium, and 50 µg/ml streptomycin. Because of the cytotoxicity of the SeV-F gene product, the gene is cloned into the pCALNdLw, where the expression of a cloned gene is inducible by Cre DNA recombinase. The above pCALNdLw/F is used for transfecting LLC-MK2 cells by the calcium phosphate method (mammalian transfection kit (Stratagene)) according to the standard protocol.

LLC-MK2 cells grown in 10-cm plates to be 40% confluent are transfected with 10 µg pCALNdLw/F and incubated in 10 ml of MEM containing 10 % FBS at 37°C under 5% CO₂ for 24 hr. Then, cells are dispersed, resuspended in 10 ml of culture medium, and plated onto five 10-cm dishes, where 5 ml of cell suspension is plated onto one dish, 2 ml onto two, and 0. 2 ml onto two. Cells are cultured in 10 ml of MEM containing 10% FBS plus 1200 µg/ml G418 (GIBCO-BRL) for 14 days with medium changed every two days, and stable transfectants are selected. Cells grown in the medium that are resistant to G418 are recovered using cloning rings. Cells of each clone are further cultured until they grow to be 100% confluent in a 10-cm dish.

To induce F protein expression, cells are grown to be 100% confluent in 6 cm dishes, and infected with AxCANCre adenovirus at moi = 3 according to the method by Saito et al. (Saito et al., Nucleic Acids Res., 1995, 23, 3816-3821; Arai T. et al., J. Virol., 1998, 72, 1115-1121).

### (3) Reconstitution and propagation of the F gene-deleted SeV virus

The pSeV18⁺/ΔF into which a foreign gene has been inserted is transfected into LLC-MK2 cells as follows. Cells are plated at 5 x 10⁶ cells/dish onto 100-mm petri dishes, cultured for 24 hr, and then infected at room temperature for 1 hr with the recombinant vaccinia virus that expresses T7 RNA polymerase and that has been treated with psoralen and long UV (365 nm) for 20 min (Fuerst T. R. et al., Proc. Natl. Acad. Sci. USA, 1986, 83, 8122-8126) (moi = 2 to 3; preferably moi = 2). UV exposure may be performed using UV Stratalinker 2400 equipped with five 15-watt bulbs (catalogue number 400676 (100 V), Stratagene, La Jolla, CA, USA). After cells are washed three times, plasmids pSeV18⁺/ΔF-GFP, pGEM/NP, pGEM/P, and pGEM/L (Kato A. et al., Genes Cells, 1996, 1, 569-579) are resuspended with OptiMEM (GIBCO) at a ratio of 12 µg/dish, 4 µg/dish, 2 µg/dish, and 4 µg/dish, respectively and mixed with SuperFect transfection reagent (5 µl SuperFect (QIAGEN) for 1 µg DNA). The mixture is incubated for 10 min at room temperature, then resuspended with 3 ml of OptiMEM with a final concentration of 3% FBS, and added to the cells. After a 3-hr culture in an incubator, cells are washed twice with serum free MEM, and further cultured in MEM containing 40 µg/ml of cytosine β-D-arabinofuranoside (AraC, Sigma) and 7.5 µg/ml of trypsin (GIBCO) for 70 hr. Then, cells are collected and resuspended in OptiMEM at 10⁷ cells/ml. Cells are frozen-thawed three times, then mixed with lipofection reagent DOSPER (Boehringer Mannheim) (10⁶ cells per 25 µl DOSPER), incubated at room temperature for 15 min, and transfected into LLC-MK2/F7 cells (10⁶ cells/well in 12-well-plate), which_ is one of the clones of F gene-expressing helper cells selected as described above. Cells are cultured in serum free MEM containing 40 µg/ml of AraC and 7.5 µg/ml of trypsin, and the culture supernatant is collected.

In preparing deletion virus vectors, two types of vectors in which the envelope gene deficient from the viral genome is different are transferred to the same cell. In this case, the envelope protein deficient in one vector is supplied by the expression of the other vector to complement each other, thereby leading to the formation of infectious virus particles and activation of the replication cycle to amplify the viral vectors. That is, when two or more types of vectors are inoculated to cells in combinations so as to complement each other's envelope proteins, mixtures of virus vectors deficient in respective envelope genes can be produced on a large scale and at a low cost. Due to the deficiency of envelope genes, these viruses have a smaller genome size compared to the complete virus, so they can harbor a long foreign gene. Also, since these originally non-infectious viruses are extracellularly diluted, and it is difficult to retain their coinfection, they become sterile, which is advantageous in managing their release to the environment.

If a vector is prepared using as the foreign gene a therapeut ic gene for a specific disease, gene therapy can be done by administering this vector. The virus vector of the present invention enables the expression of a foreign gene that can help cure a disease, an endogenous gene that is lacking or insufficient in patients, either through direct administration or indirect administration (*ex vivo*). A foreign gene is not limited, and may include those encoding a secretory protein that is desirably secreted into the cardiovascular system. Humoral factors such as various cytokines, hormones, growth factors, and the like are examples. It is preferable that an anti-inflammatory cytokine gene is used as the foreign gene for treating inflammatory diseases.

Recovered paramyxovirus can be purified so as to be substantially pure. Purification can be performed by known purification and separation methods including filtration, centrifugation, column chromatographic purification, and the like or by combination thereof. The term "substantially pure" used herein means that the virus occupies the main ratio as a component of the sample in which the virus exists. Typically, substantially pure virus vectors can be detected by confirming that the ratio of the virus-derived proteins to the total proteins in the sample occupies 50% ormore, preferably 70% ormore, more preferably 80% or more, and even more preferably 90% or more. Specifically, paramyxovirus can be purified, for example, by a method in which cellulose sulfate ester or crosslinked polysaccharide sulfate ester is used (Examined Published Japanese Patent Application (JP-B) No. Sho 62-30752; JP-B Sho 62-33879; JP-B Sho 62-30753), a method in which adsorption to fucose sulfate-containing polysaccharide and/or a decomposition product thereof is used (WO97/32010), etc.

The paramyxovirus vector can be made as a composition together with a desired, pharmaceutically acceptable carrier. Herein, a "pharmaceutically acceptable carrier" is defined as a material that can be administered with a vector, but does not inhibit gene transfer by the vector. Forinstance, the paramyxovirus vector may be appropriately diluted with saline, phosphate buffered saline (PBS), and the like to prepare a composition. When the paramyxovirus vectors of this invention are proliferated in chicken eggs and the like the composition can include allantoic fluid. Compositions comprising the paramyxovirus vectors of this invention may contain physiologically acceptable media such as deionized water, 5% dextrose aqueous solution, and the like, and, furthermore, other stabilizers and antibiotics may also be contained. The composition can be used as a pharmaceutical composition. Thus, the present invention also relates to the use of the vector or the above composition as a pharmaceutical as described above.

Specifically, the composition used in accordance with the present invention includes:
(1) a composition for the treatment of inflammatory diseases comprising the paramyxovirus vector disclosed herein, or cells containing said vector, wherein the gene product of said vector is introduced to a site different from the site of injection through the blood stream;
(2) the composition for the treatment of inflammatory diseases of (1), wherein said vector contains a foreign gene;
(3) the composition of (2), wherein said foreign gene is a cytokine gene;
(4) the composition of (3), wherein said cytokine is an anti-inflammatory cytokine;
(5) the composition of (4), wherein said anti-inf lammatory cytokine is IL-10;
(6) the composition of (1) and (5), wherein said inflammatory disease is pulmonary fibrosis;
(7) the composition of any of (1) to (6), which is administered intramuscularly; and
(8) the composition of any of (1) to (7), wherein said paramyxovirus is Sendai virus.

The above paramyxovirus vector, or a composition comprising said vector is administered to transduce a foreign gene harbored within the vector in accordance with the definition of the claims.

The site of administration is intramuscular administration (IM). The vector may be administered *in vivo* or *ex vivo,* and to a single site or multiple sites. In addition, the vector may be administered only once or multiple times.

The gene transferred by the paramyxovirus vector is not limited as long as it encodes a secretory protein, but maybe a gene encoding a variety of cytokines or hormones. These proteins may include members of their respective families, and isoforms as well. The paramyxovirus vector is particularly useful for expressing, in the whole cardiovascular system, therapeutic genes of biologically active substances and the like having a short half-life in blood (such as cytokines). The half-life of a natural cytokine in the cardiovascular system is generally very short. For instance, natural IL-10 is only effective for the initial 30 min after administration (Gerard et al., J. Exp. Med., 1993, 177 (2), 547). Use of the paramyxovirus vector of the invention enables a continuous supply of biologically active substances with a short half-life, including cytokines, to the blood for a long period of time.

In particular, cytokines have a broad range of functions related to cell proliferation and differentiation. For instance, IL-1 has a variety of functions including T cell activation, expression of the IL-2 receptor, induction of cytokine genes and others, regulation of cell proliferation, and regulation of hormone secretion. IL-1 stimulates the release of cell growth and differentiation factors from bone marrow and lymphocyte cell lines. In addition, IL-1 induces the proliferation of pluripotent progenitors in bone marrow and promotes hematopoiesis. It is effectively used in cancer therapy. Also, IL-1 is involved in angiogenesis and activation of fibroblasts, and is also useful in wound healing.

IL-2 is involved in the activation of T-, NK-, and LAK cells, growth promotion of specific types of cells, enhancement of the production of immunoglobulin and IFN-γ, induction of IL-6 production from monocytes, etc. IL-2 is effectively used for the treatment of tumors and immunodeficiency diseases, or activation of NK cells after bone marrow transplantation. IL-3 is involved in mast cell proliferation, production of blood cells from hematopoietic progenitor cells, etc. It functions in concert with other cytokines (IL-6, for instance) to regulate differentiation of a variety of blood cells. IL-3 can be used effectively in the treatment of, for example, aplastic anemia.

IL-4 is involved in the increase in expression of the MHC class II gene in resting B cells, proliferation of activated T-cells, and promotion of effector cell functions. IL-4 is also involved in mast cell proliferation, promotion of cell maturation in the thymus, proliferation of blood cells, etc. IL-4, in the presence of IL-1, enhances antigen presentation and phagocytosis in macrophages. IL-4 participates in the functional reconstitution of the cellular and humoral immune systems after bone marrow transplantation, cures immunodeficiency with increased IgM, induces terminal differentiation in acute lymphoblastic leukemia, inhibits growth of solid tumors and B cell lymphoma, suppresses inflammation through inhibition of production of IL-1, TNF, and IL-6, etc. In addition, IL-4 is expected to be useful in the treatment of T cell dependent autoimmune diseases such as autoimmune diabetes and allergic encephalomyelitis, and other diseases in which the T cell dependent immune function is involved (Rapoport et al., J. Exp. Med., 1993, 178, 87-99; Racke et al., J. Exp. Med-, 1994, 180, 1961).

IL-5 is effective in the stimulation of IgA production and growth promotion of eosinophilic leukocytes, and is known for its application in the treatment of schistosomiasis (Sanderson, "International Conference on the Clinical Impact of Interleukins" at the Royal College of Physicians in London, 1989 April), and tumors (Kolb et al., Br. J. Cancer, 1979, 40, 410; Pretlow et al., Cancer Res., 1983, 43, 2997; Iwasaki et al., Cancer, 1986, 58, 1321). Furthermore, IL-6 is reported to have many functions including the induction or inhibition of proliferation of a variety of cells. IL-7 induces proliferation of a particular type of B cells and T cells. IL-9 is involved in erythrocyte differentiation, T cell survival, immunoglobulin production from B lymphocytes, and the stimulation of IL-6 production from mast cells.

IL-10 is produced by activated Th2 cells, B cells, keratinocytes, monocytes, and macrophages (Moore et al., Annu. Rev. Immunol., 1993, 11, 165). IL-10 is effective in the stimulation of proliferation and differentiation of B cells. In addition, IL-10 represses cytokine production (such as IFN-γ, TNF-β, and IL-2) by Th1-cells, NK-cells, monocytes, and macrophages (Fiorentino et al., J. Exp. Med., 1989, 170, 2081-2095; Fiorentino et al., J. Immunol., 1991, 146, 3444; Hsu et al., Int. Immunol., 1992, 4, 563; D'Andrea et al., J. Exp. Med., 1993, 178, 1041; de Waal Malefyt et al., J. Exp. Med., 1991, 174, 915; Fiorentino et al., Int. Immunol., 1991, 147, 3815). Accordingly, IL-10 is effective, through the suppression of the Th1-cell reaction, in preventing rejection after transplantation, and T-cell mediated autoimmune diseases such as type I diabetes and multiple sclerosis. IL-10 inhibits the secretion of pro-inflammatory cytokines (such as IL-1, IL-6, IL-8, and TNF-α). Therefore, IL-10 may be used for the treatment of inflammatory diseases including rheumatoid arthritis and psoriasis. Because IL-10 inhibits the secretion of tumor necrosis factor and suppresses septic shock, it may be useful in the treatment of sepsis. IL-10 may also be useful in the suppression of granuloma formation due to schistosomes and the suppression of liver fibrosis as well. Interferon-α and β have antiviral functions against the papilloma virus, hepatitis virus, herpes virus, and so on, and may be useful for the treatment of hairy cell leukemia, myeloma, and other tumors in hematopoietic organs.

To transfer the above described cytokines to the cardiovascular system, the paramyxovirus vector of the present invention expressing those cytokines may be suitably used. In one desirable embodiment, the paramyxovirus vector contains a gene encoding an anti-inflammatory cytokine. Anti-inflammatory cytokines may include, for example, IL-10, IL-4, and IL-12. The paramyxovirus vector that expresses anti-inflammatory cytokines can be a useful anti-inflammatory drug.

The vector disclosedherein can be applicable to gene therapy of various diseases. Such gene therapy may include the treatment of disease accompanying inflammation, including, pulmonary fibrosis, sclerosing peritonitis, prostatomegaly, multiple sclerosis, post transplantation rejection, type I diabetes, rheumatoid arthritis, inflammatory enteropathy, psoriasis, systemic lupus erythematosus, iritis, granulomatous diseases, chronic nephritis, scleroderma, hysteromyoma, keloid, cirrhosis, and other diseases accompanying inflammation. The vector may also be useful in the creation of various disease animal models, and also in the development or evaluation of methods for treating those disease models and such.

For instance, the vector disclosed herein may be preferably used in the gene therapy of pulmonary fibrosis. Pulmonary fibrosis is a disease with fibrosis of lung including chronic interstitial pneumonitis, where, in many cases, the cause of the disease is unclear, but is accompanied by lung fibrosis caused by either chronic infectious diseases or an abnormal autoimmune system. Specifically, the disease includes pneumonitis and cystic fibrosis (CF).

The paramyxovirus vector disclosed herein may be administered at a sufficient dose so that an effective dose of vectors can be transferred to the cells of the target tissue. Herein, the "effective dose" is defined as a dose that enables the introduction of genes to the cells of the target tissue so as to bring, at least partially, the desired therapeutic effect or preventive effect. The administration of an effective dose of the paramyxovirus vector containing a desired gene enables the transfected cells to produce the gene product. Preferably, the administration of an effective dose of the paramyxovirus vector containing a desired gene may allow the detection of a significant level of expression of the transfected gene in the administered tissue or in blood. A "significant level" is defined as the level at which the expression of the transfected gene (the amount of transcripts or translated products) is detectable. For instance, if there is an endogenous counterpart of the transfected gene, the maximal level of the transfected gene must be significantly higher than the expression level of the endogenous one. The expression of the transfected gene may be approximately 1.2 fold or more, preferably about 1.5 fold or more, more preferably about 2 fold or more, much more preferably about 10 fold or more, and most preferably about 20 fold higher than the expression level of the endogenous gene at the site of administration or in blood. However, the expression level of the transfected gene must be determined by considering its effective level and toxic level.

The expression level of genes transfected into cells can be determined by assays known to those skilled in the art. Transcripts may be detected and quantified by Northern hybridization, RT-PCR, RNA protection assay, and the like. Detection by Northern hybridization, RT-PCR, and the like may be performed *in situ.* To detect translated products, western blot, immunoprecipitation, RIA, ELISA, pull down assay, and the like may be adopted using antibodies. For an easy detection of transfected gene products, the protein to be expressed may be tagged, or a reporter gene may be contained in the vector. The reporter gene may be that encoding β-galactosidase, chloramphenicol acetyltransferase (CAT), alkaline phosphatase, or green fluorescent protein (GFP), but is not limited to these.

The dose of the vector used for administration may vary depending on a disease, body weight, age, sex, symptom, administration purpose, and gene to be introduced, and the like, but it can be properly determined by those skilled in the art. It is preferable to inoculate, with pharmaceutically acceptable carriers, the vectors whose dose is within the range of preferably about 10⁵ pfu/ml to about 10¹¹ pfu/ml, more preferably about 10⁷ pfu/ml to about 10⁹ pfu/ml, and most preferably about 1 x 10⁸ pfu/ml to about 5 x 10⁸ pfu/ml. The subj ect of inoculation of the compositions containing paramyxovirus vectors includes all mammals such as humans, monkeys, mice, rats, rabbits, sheep, bovines, dogs, etc.

### Brief Description of Drawings

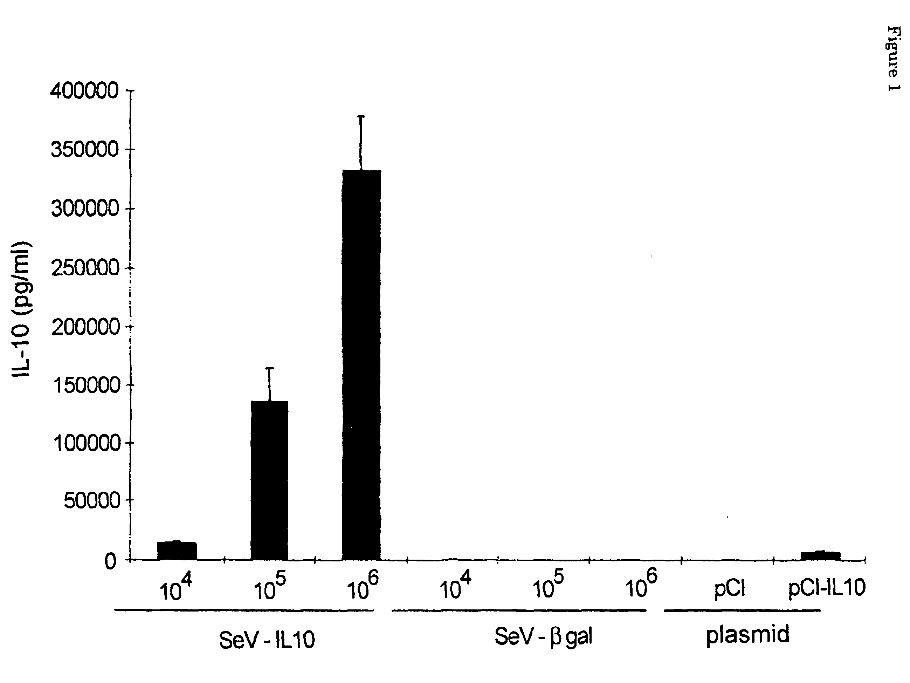

Figure 1 shows the dose-dependent expression of IL-10 in SeV-IL10-transfected COS7 cells in 24-well plates. COS7 cells were infected with SeV-IL10 at the dose indicated, and after 16 hr, the concentration of IL-10 secreted into the culture medium was measured. As a control, cells were transfected with a plasmid DNA for expressing IL-10. For each group, n = 6.

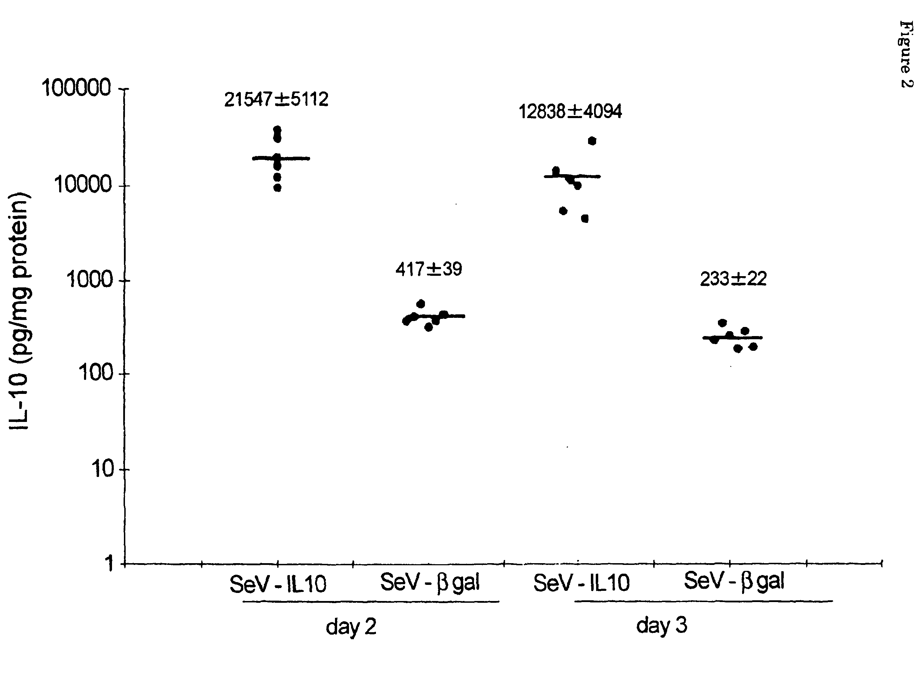

Figure 2 shows the expression of IL-10 in the lung of mice that were intranasally given SeV-IL10. SeV-IL10 (6.8 x 10⁷ pfu/100 µl) was intranasally administered by sniffing. Two days after administration, the mice were sacrificed and the expression level of IL-10 in lung homogenates was quantified. The IL-10 expression in control mice that were given the IL-10 expression plasmid was 310±54 pg/mg protein (mean ± SEM).

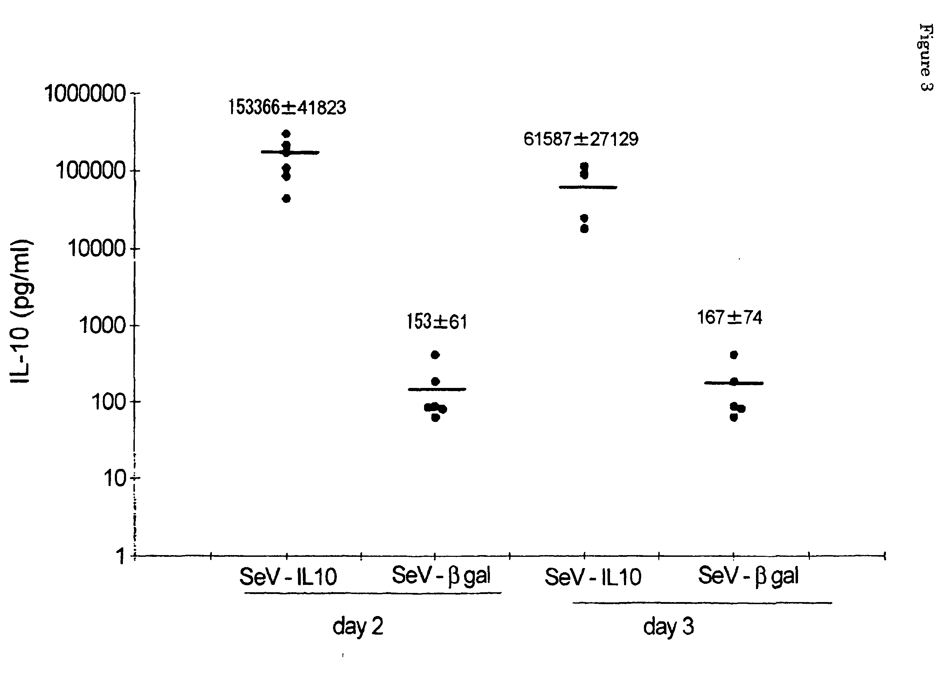

Figure 3 shows the secretion of IL-10 into BALF of mice that were given SeV-IL10 intranasally. SeV-IL10 (6.8 x 10⁷ pfu/100 µl) was intranasally administered by sniffing. Two days after administration, the mice were sacrificed, and the IL-10 expression level in BALF was determined. The IL-10 expression in control mice that were given the IL-10 expression plasmid was 71±63 pg/ml (mean ± SEM).

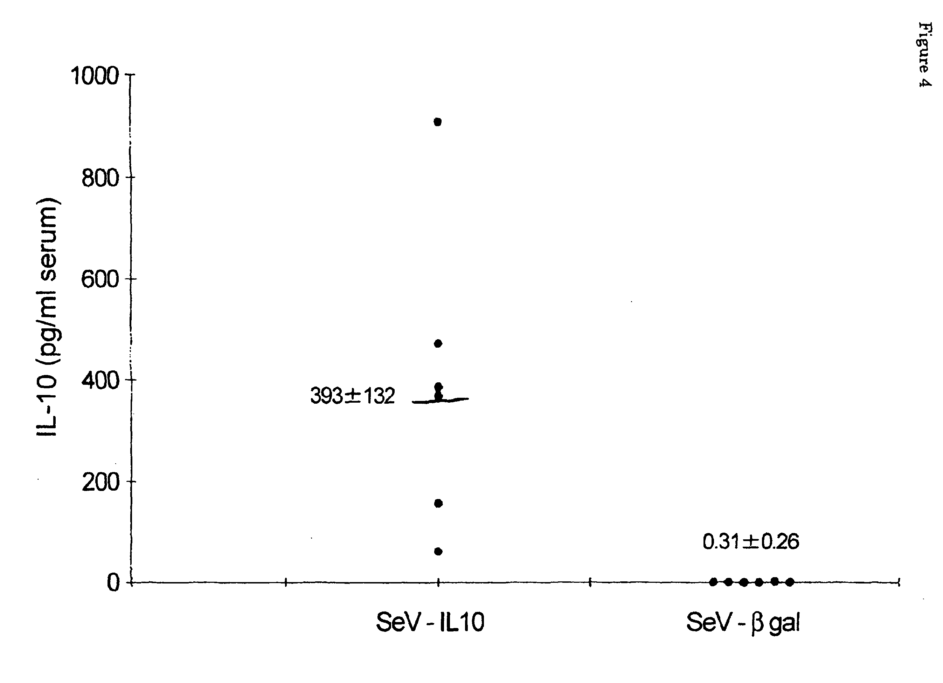

Figure 4 shows the secretion of IL-10 into blood in mice that were intranasally given SeV-IL10. SeV-IL10 (6.8 x 10⁷ pfu/100 µl) was intranasally administered by sniffing. Two days after administration, the mice were sacrificed, and the IL-10 level in serum was determined.

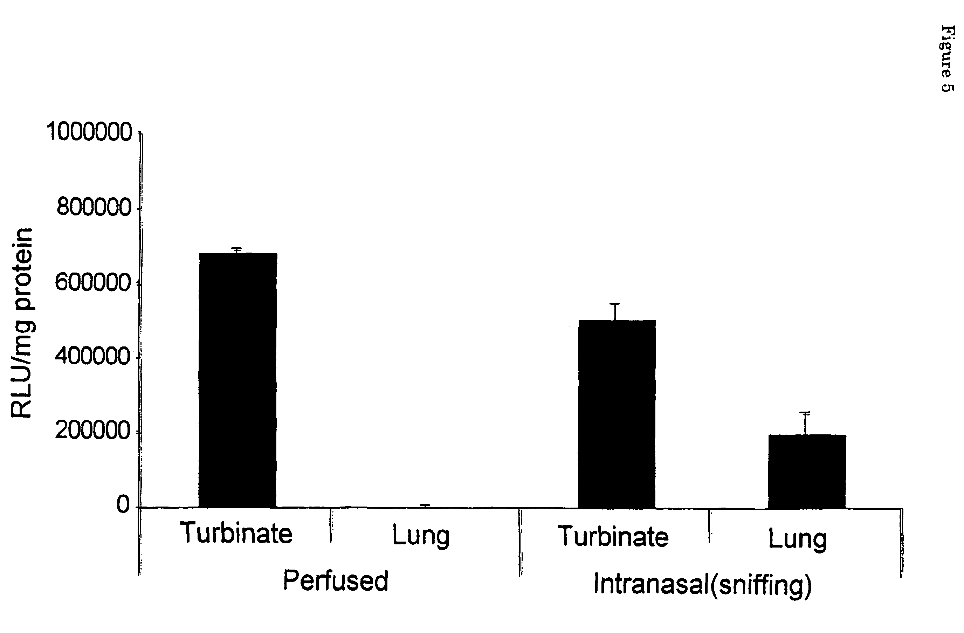

Figure 5 shows the expression of transfected genes in the turbinate and lung by administration of the Sendai virus vector by sniffing or perfusion. SeV-luc (7 x 10⁷ pfu/100 µl) was administered by sniffing or perfusion to nasal epithelia (turbinate), and after 48 hr, the mice were sacrificed and the expression of luciferase in the lung and the turbinate was examined (n = 3).

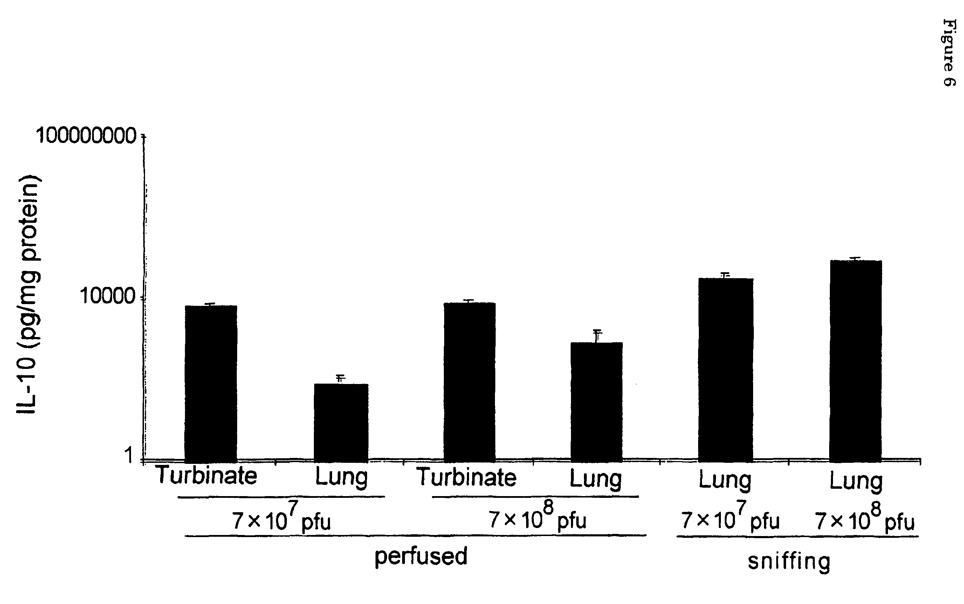

Figure 6 shows the expression of IL-10 in the turbinate and lung by administration of SeV-IL10 by sniffing or perfusion. SeV-IL10 (7 x 10⁷ pfu/100 µl and 7 x 10⁸ pfu/100 µl) was administered by sniffing or perfusion to nasal epithelia (turbinate), and after 48 hr, the mice were sacrificed and the IL-10 expression in the lung and turbinate was examined (n = 6).

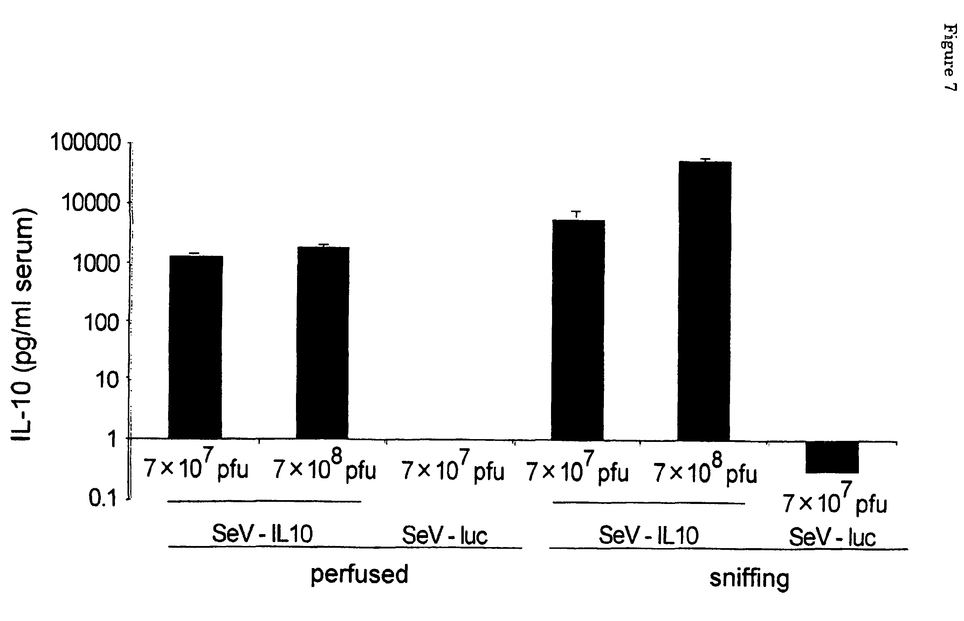

Figure 7 shows the secretion of IL-10 into blood by administration of SeV-IL10 by sniffing or perfusion. SeV-IL10 (7 x 10⁷ pfu/100 µl and 7 x 10⁸ pfu/100 µl) was administered by sniffing or perfusion to nasal epithelia (turbinate), and after 48 hr, mice were sacrificed and the IL-10 level in serum was examined (n = 6).

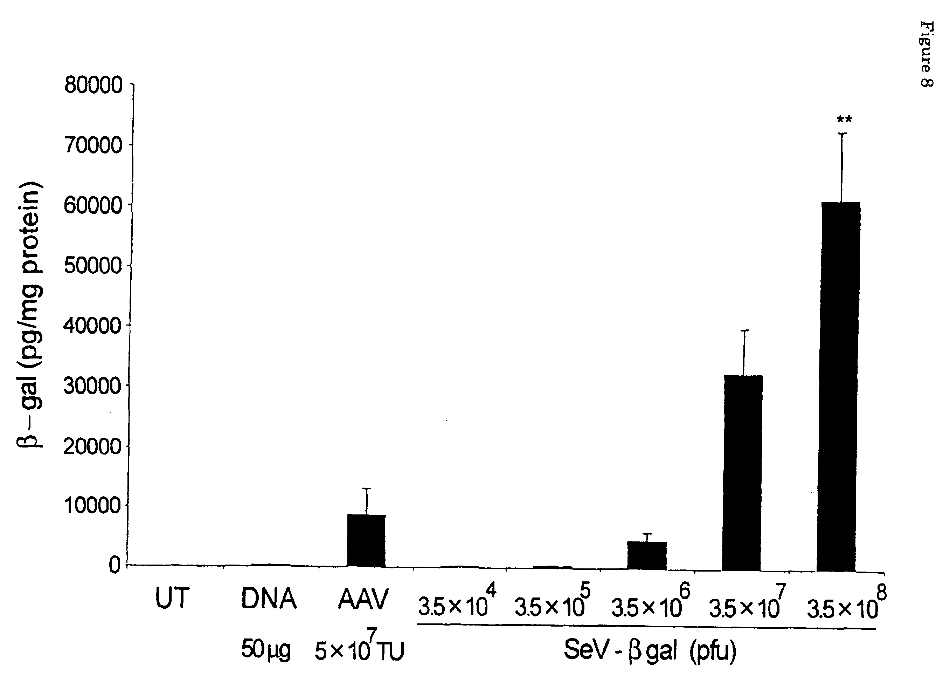

Figure 8 shows the dose-dependent expression of β-galactosidase after injection of SeV-βgal into the tibialis anterior (TA) muscles. Mice were injected into each TA muscle with increasing amounts of SeV-βgal, or plasmid DNA or AAV expressing the β-galactosidase reporter gene or remained uninjected (UT). Virus dose and amount of DNA represents the amount injected into each TA. Two days after injection, the mice were sacrificed and the expression level of β-galactosidase in the muscle homogenate was determined. Data are represented as mean ± SEM. For each group, n = 6 to 12. ** p<0.01 in comparison with DNA-injected group and AAV-injected group.

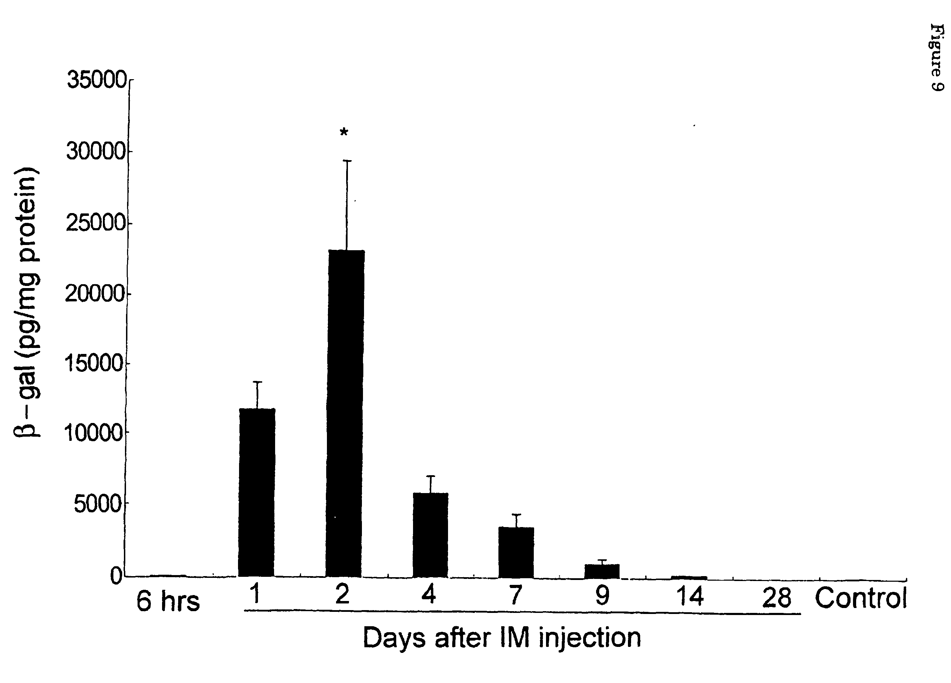

Figure 9 shows the time course of β-galactosidase expression after injection of SeV-βgal into the tibialis anterior (TA) muscles. Mice were injected into each TA muscle with SeV-βgal (3.5 x 10⁷ pfu/muscle) or SeV-luciferase (3.5 x 10⁷ pfu/muscle) as control. After injection, mice were sacrificed at the indicated time, and the expression level of β-galactosidase in the muscle homogenate was determined. Mice injected with the control virus were sacrificed two days after injection. Data are represented as mean ± SEM. For each group, n = 6. * p<0.05 in comparison with the value at 4 days after injection.

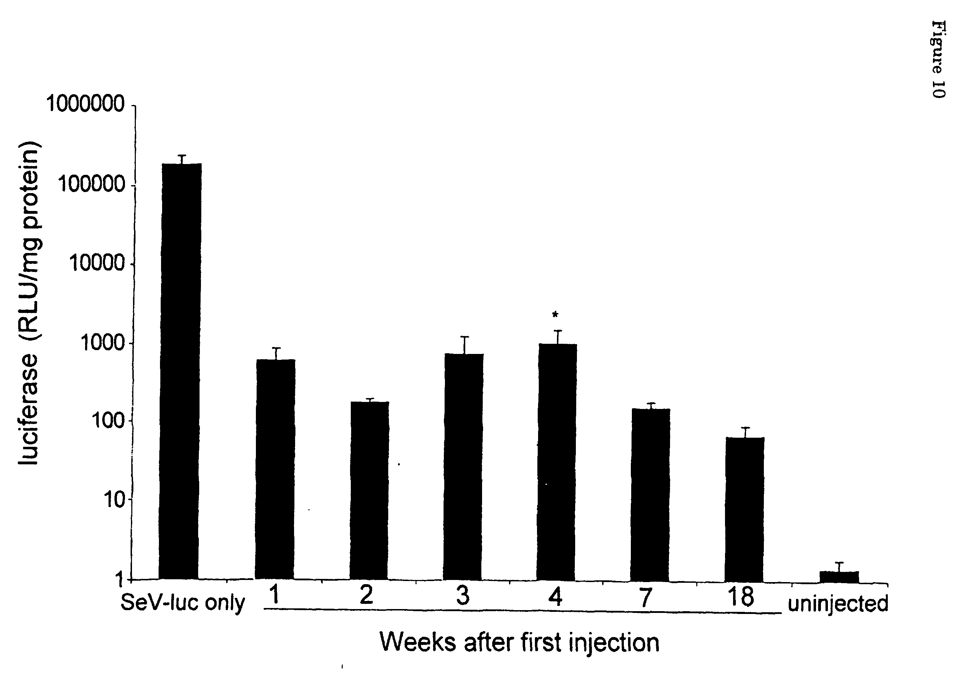

Figure 10 shows the effect of repetitive injection of Sendai virus vector into muscles. Mice were injected into each TA muscle with SeV-βgal (3.5 x 10⁷ pfu/muscle) and received subsequent injections with SeV-luciferase (3.5 x 10⁷ pfu/muscle) at indicated time-points. Two days after the second injection, the mice were sacrificed, and the luciferase expression in the muscle homogenate was examined. The expression level of luciferase in each mice was compared to mice receiving only one injection of SeV-luciferase or uninjected mice. Data are represented as mean ± SEM. For each group, n= 6 to 10. * p<0.05 in comparison with the value in untreated mice.

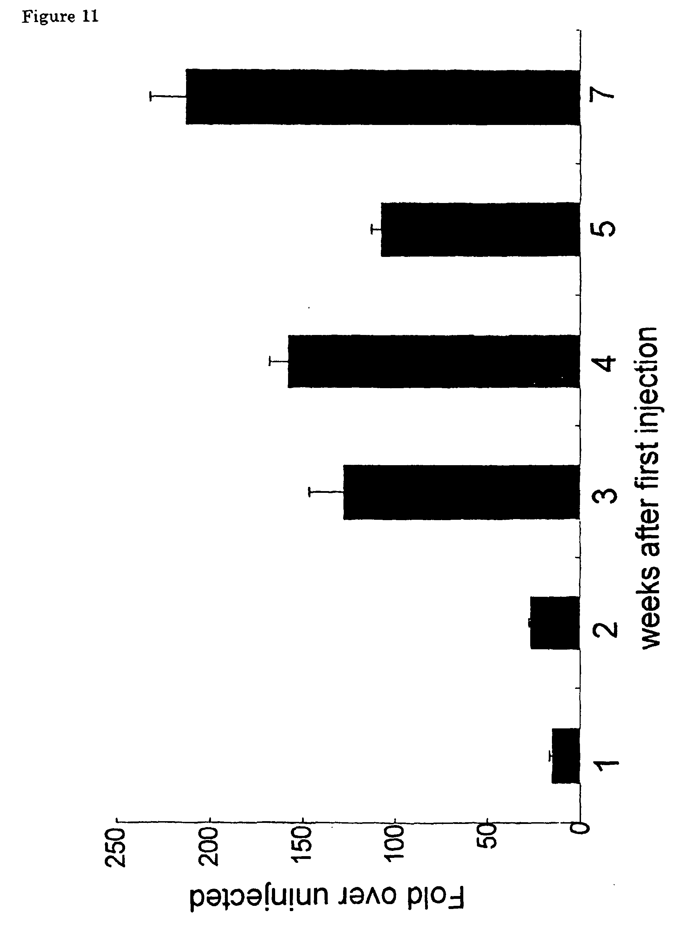

Figure 11 shows the results of examination for the presence of systemic anti-SeV antibody after intramuscular injection of the Sendai virus vector. Mice were injected into each TA muscle with SeV-βgal (3.5 x 10⁷ pfu/muscle). After injection, the mice were sacrificed at the indicated time, and the presence of antibodies specific to SeV in serum was examined. Data are represented as mean ± SEM. For each group, n = 6.

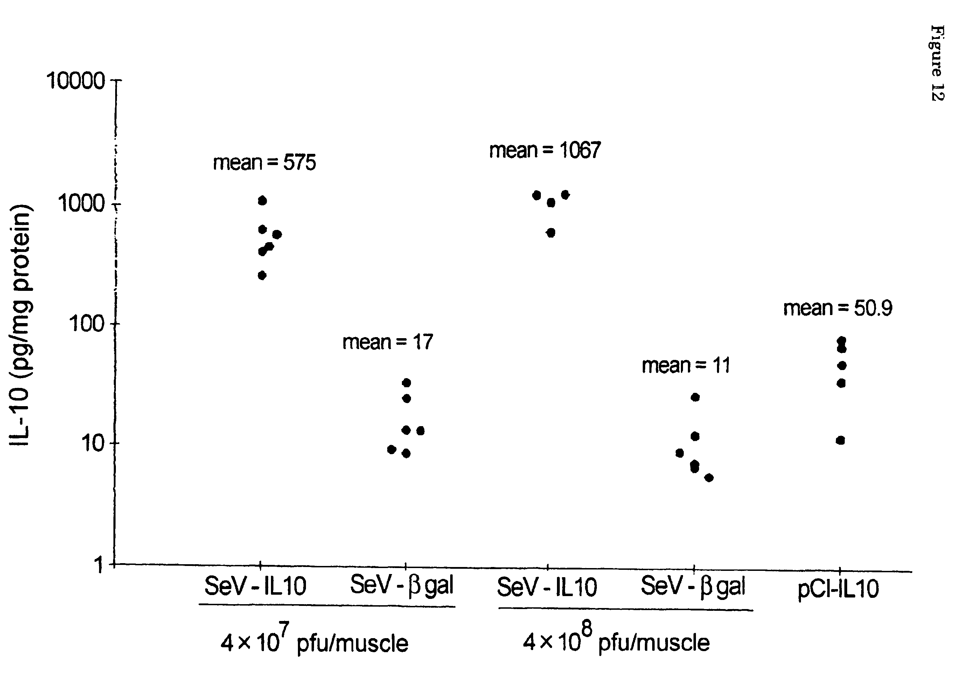

Figure 12 shows the expression of IL-10 in muscle after intramuscular injection of SeV-IL10. Mice were injected into each TA muscle with SeV-IL10 at the indicated dose, and after two days, the mice were sacrificed and IL-10 level in the muscle homogenate was measured. As a control, plasmid DNA for IL-10 expression (pCI-IL10) or SeV-βgal was injected.

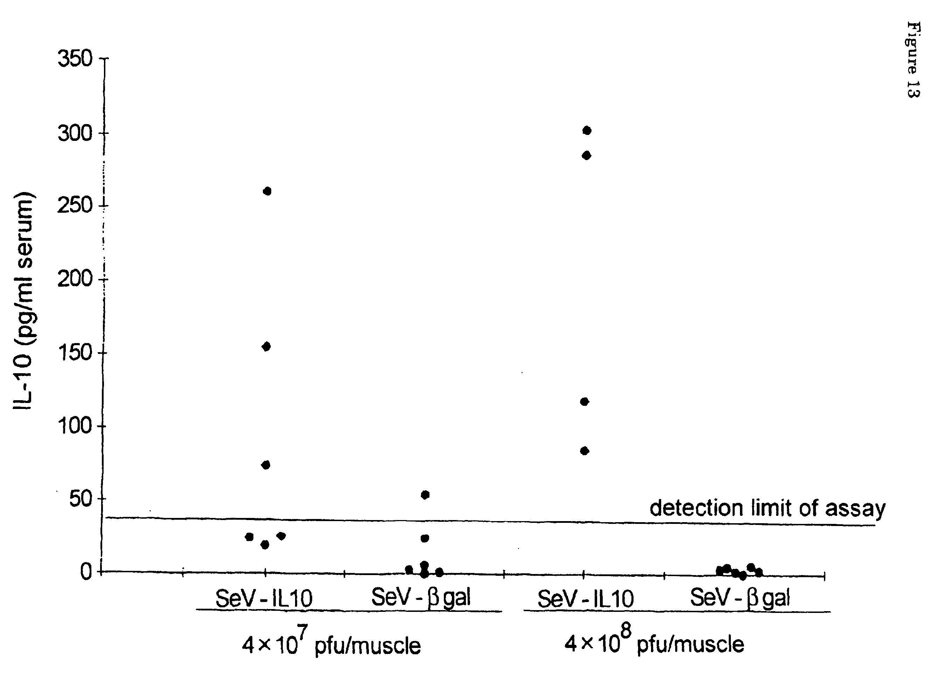

Figure 13 shows the secretion of IL-10 to blood after intramuscular injection of SeV-IL10. Mice were injected into each TA muscle with SeV-IL10 at the indicated dose, and after two days, the IL-10 level in serum was determined. Plasmid DNA for IL-10 expression (pCI-IL10) or SeV-βgal was injected as controls. The IL-10 level in the serum of mice injected with plasmid DNA for IL-10 expression was below the limit of detection. (n = 4 to 6)

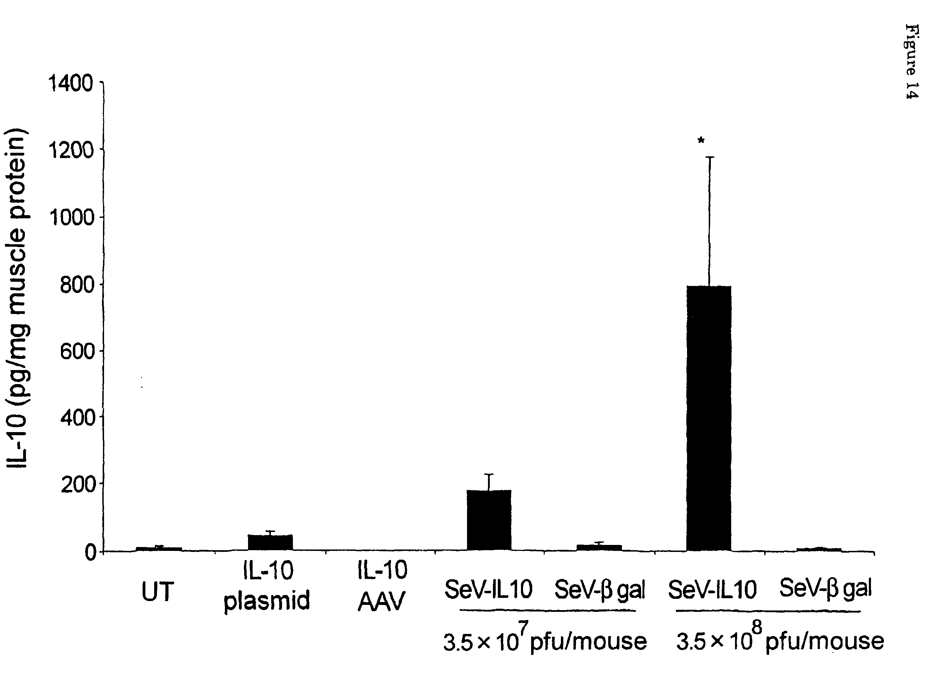

Figure 14 shows the overexpression of IL-10 in muscle after intramuscular injection of SeV-IL10. Mice were injected into each TA muscle with SeV-IL10 (3.5 x 10⁷ or 3.5 x 10⁸ pfu/muscle), or with plasmid DNA or AAV (IL10AAV) for IL-10 expression. Untreated mice were also examined. Two days after injection, the mice were sacrificed and the IL-10 level in the muscle homogenate was examined. Data are represented as mean ± SEM. For each group, n = 6. * p<0.05 in comparison with all the other groups.

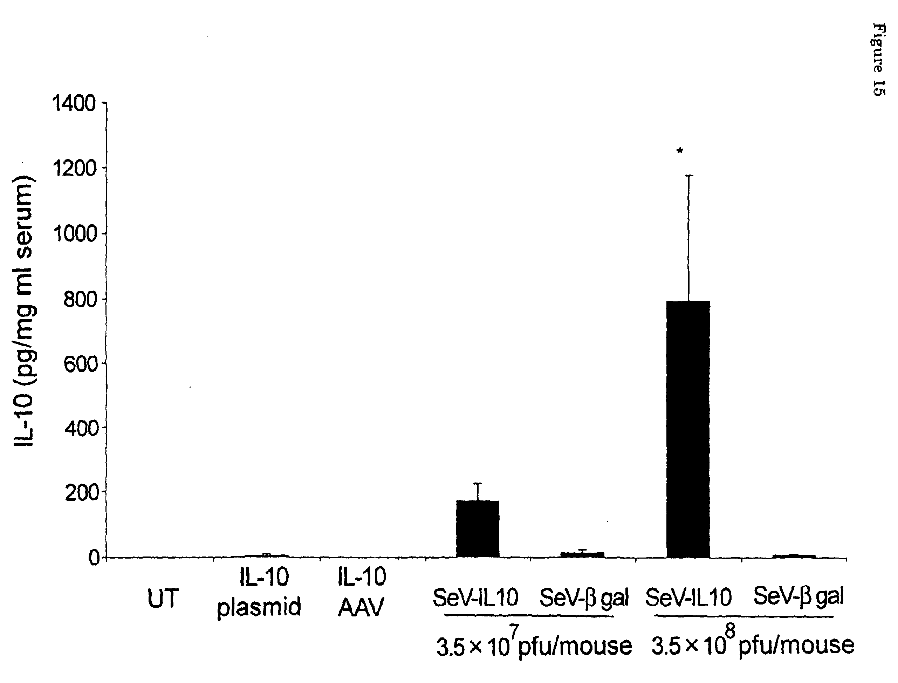

Figure 15 shows the secretion of IL-10 into blood after intramuscular injection of SeV-IL10. Mice were injected into each TA muscle with SeV-IL10 (3.5 x 10⁷ or 3.5 x 10⁸ pfu/muscle), or with plasmid DNA or AAV (IL10AAV) for IL-10 expression. Untreated mice were also examined. Two days after, the mice were sacrificed, and the IL-10 level in serum was determined. Data are represented as mean ± SEM. For each group, n = 6. * p<0.05 in comparison with all the other groups.

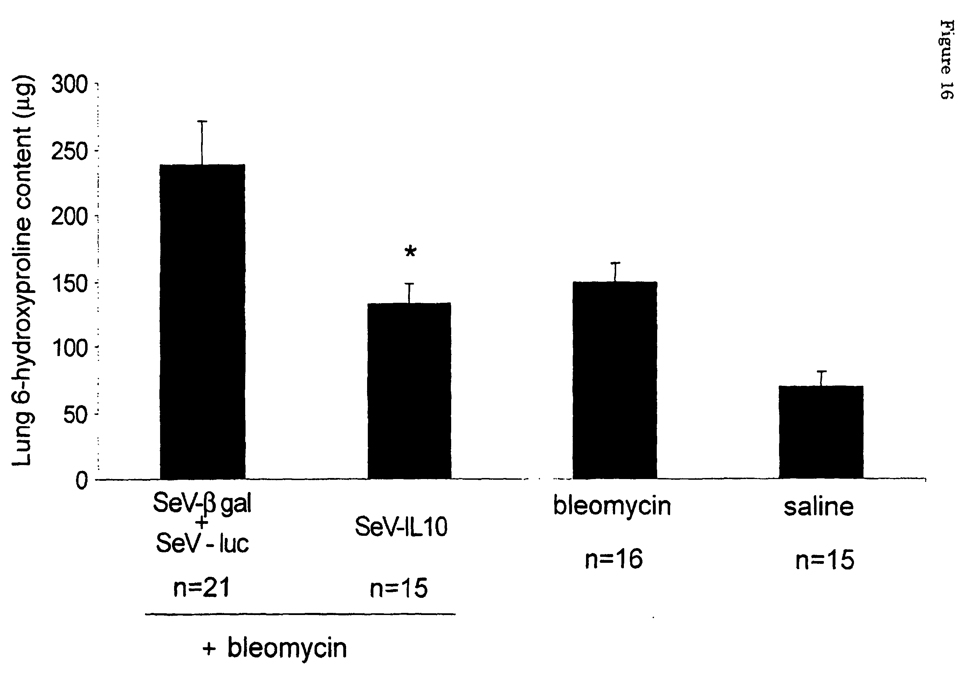

Figure 16 shows the effect of intramuscular injection of SeV-IL10 in pulmonary fibrosis model mice. Mice were injected into each TA muscle with SeV-IL10, or with SeV for luciferase expression and SeV for β-galactosidase expression used as controls (7 x 10⁸ pfu/mouse each). Two days after injection, mice were recieved in intratracheal injection of bleomycin (0.075 U/100 µl) or saline. Eleven days after bleomycin injection, the mice were sacrificed and collagen deposition in lung was examined by the hydroxyproline assay. For each group, n = 15 to 21. * p<0.05 in comparison with the control group injected with SeV-luciferase/β-gal.

### Best Mode for Carrying Out the Invention

The present invention is illustrated in detail below with reference to examples, but it is not to be construed as being limited thereto. All the references cited herein are incorporated by reference.

### Example 1: In vivo administration of Sendai virus vector

Recombinant Sendai virus vectors containing a gene encoding IL-10, β-galactosidase, or luciferase (SeV-IL10, SeV-βgal, and SeV-luciferase, respectively) that are capable of replication were constructed according to the method described in Kato A. et al., EMBO J., 1997, 16, 578-587 and Yu D. et al., Genes Cells, 1997, 2, 457-466. The vectors were propagated in chicken eggs; and the allantoic solution containing viruses were stored at -80°C until use as a composition comprising the recombinant Sendai virus vector of the present invention.

Sendai virus vectors were administered in the examples as follows.

### 1. Intranasal administration

Mice were anesthetized by metophane inhalation, and the vector was administered by sniffing. Unless otherwise specified, a dose of 7 x 10⁶ to 7 x 10⁸ pfu of SeV-IL10 or control virus SeV-βgal in a total 100 µl volume was inoculated to the nose of the mouse and were sniffed in as a bolus over a 10 to 20 second period. This method resulted in virus deposition in the lung and in the nose.

### 2. Perfusion

Mice were anesthetized with hipnom/hipnoval. SeV-IL10 or control virus SeV-βgal was applied selectively to the nasal epithelium (turbinate) over a 10 minute period by administering the virus through a thin catheter directly onto the nasal epithelium at 7 x 10⁷ or 7 x 10⁸ pfu in a total 100 µl volume, unless otherwise specified. During the procedure, the mouse was kept in an inverted position with its head down. Excess virus therefore dripped out of the nose. This method resulted in virus deposition predominantly in the nose, very little virus reached the lung.

### 3. Intramuscular administration

Unless otherwise specified, C57BL/6 mice were injected with 7 x 10⁷ or 7 x 10⁸ pfu of SeV-IL10 or control SeV-βgal into both tibialis anterior (TA) muscles (50 µl per each muscle, n = 6 to 11). A dose-dependent response was observed in a pilot experiment using SeV-βgal (from 7 x 10⁴ to 7 x 10⁹ pfu/animal).

### Example 2: Preparation of tissue lysates, sera, and broncho-alveolar lavage fluid (BALF)

Mice were sacrificed 48 hr after virus administration. Tissue lysates, sera, and BALF were prepared as follows.

### 1. Tissue lysates

TAmuscles, lung, and turbinate were excised and homogenized in 300 µl of 0.25 M Tris-HCl (pH 8) solution. Cells were lyzed by three cycles of freeze-thawing. Samples were centrifuged at 14,000 rpm for 10 min, and the supernatants were stored at -80°C for subsequent analysis. The protein concentration of each sample was determined by the modified Folin-Lowry protein assay.

### 2. Sera

A blood sample was collected from the heart, and incubated at 37°C for 2 hr to induce clot formation. Samples were then centrifuged at 4,000 rpm for 10 min, and the obtained sera was transferred to a new tube and frozen at -80-°C for subsequent analysis.

### 3. BALF

The mouse trachea was exposed and a catheter was inserted. The lung was lavaged three times with 0.5ml PBS. BALFwas collected and centrifuged at 4,000 rpm for 5 min, and was frozen at -80°C for subsequent analysis.

### Example 3: Interleukin-10 (IL-10) ELISA

ELISA of human IL-10 was performed using a kit purchased from R&D according to the manufacturer's instructions. The amount of IL-10 in the tissue lysates was standardized by protein concentration, and data was calculated as the IL-10 amount in picograms per milligram of cytosolic protein.

### Example 4: Reporter gene assay

Luciferase assay was performed using a kit purchased from Promega according to the manufacturer's instructions. β-galactosidase assay was performed using a kit purchased from Clontech also according to the manufacturer's instructions. These assays were performed using tissue lysates and data was standardized by protein concentration.

### Example 5: Statistical analysis

All data were shown as mean values ± standard error (SEM). Variance was analyzed by the Mann-Whitney test, and data with p < 0.05 were considered as significant.

### Example 6: Transfection of IL-10 expressing Sendai virus vector into COS7 cells

COS7 cells were transfected with SeV-IL10 at different doses (10⁴ to 10⁶ pfu/well in 24 well plates). After 16 hr of transfection, the amount of secreted IL-10 in the culture supernatant was examined. IL-10 expressing plasmid (pCI-IL10) (80 µg/ml, 100 µl) was transiently transfected (by liopofection), and IL-10 expression was compared with that of Sev-IL10. SeV-βgal was used as a control.

In cells transfected with SeV-IL10, the amount of secreted IL-10 increased in a dose-dependent manner (Fig. 1). The amount of IL-10 secreted from the cells infected with SeV-IL10 at the highest titer (10⁶ pfu/well) was higher by two orders of magnitude than that in cells transfected with a plasmid encoding IL-10 by lipofection (SeV-IL10: 333±47 ng/ml/mg protein; plasmid: 6.8±1.4 ng/ml/mg protein).

### Example 7: Intranasal administration of Sendai virus vector into mice by sniffing

Following topical administration of the virus vectors (6.7 x 10⁷ pfu/100 µl) to the mouse airways via intranasal instillation, IL-10 secretion was measured in lung homogenates and BALF two days after transfection and compared to IL-10 secretion following liposome-mediated transfection with plasmid DNA. The concentration of IL-10 in serum was also determined.

In lung homogenates, the amount of secreted IL-10 was higher by two orders of magnitude than that obtained by plasmid transfection (SeV-IL10: 21457±5112 pg/mg protein; plasmid: 310±54 pg/mg protein) (Fig. 2). Furthermore, in BALF, the amount of secreted IL-10 after two days of infection was higher by three orders of magnitude than that obtained by plasmid transfection (SeV-IL10: 153366±41823 pg/ml; plasmid: 71±63 pg/ml) (Fig. 3). After two days of administration, the IL-10 level in serum was significantly higher in SeV-IL10 infected mice than in control virus infected mice (SeV-IL10: 393±132 pg/ml; SeV-βgal: 0.31±0.26 pg/ml) (Fig. 4).

### Example 8: Intranasal administration of Sendai virus vector by sniffing or perfusion

Virus vectors were administered by intranasal instillation (sniffing) as in Example 7, or directly administered to the nasal epithelia (turbinate) by perfusion, and the expression of the transfected gene was compared. Mice were infected with SeV-luc (7 x 10⁷ pfu/100 µl) by either sniffing or perfusion into the nasal epithelia (turbinate), and sacrificed after 48 hr, and luciferase expression in lung and turbinate was examined (n = 3). In mice infected by sniffing, a significant expression of luciferase gene was observed in both lungs and turbinate. In contrast, in mice infected by perfusion, luciferase expression was restricted to the turbinate, and was hardly detected in lungs (Fig. 5).

Next, SeV-IL10 (7 x 10⁷ pfu/100 µl and 7 x 10⁸ pfu/100 µl) was administered by sniffing or perfusion into the nasal epithelia (turbinate), and mice were sacrificed after 48 hr, and IL-10 expression in the lungs and turbinate was examined (n = 6). A significant ainount of IL-10 was detected in lungs of mice to whom the virus vectors were administered by sniffing. Although a significant expression was also detected in turbinate homogenates of mice infected by perfusion, only a trace level of IL-10 expression was detected in their lung homogenates (Fig. 6).

The secreted IL-10 level in serum was determined in the above mice administered with SeV-IL10. SeV-IL10 (7 x 10⁷ pfu/100 µl and 7 x 10⁸ pfu/100 µl) was administered by sniffing or perfusion into the nasal epithelia (turbinate), and mice were sacrificed after 48 hr, and IL-10 expression in serum was examined (n = 6). High level IL-10 secretion was observed in serum of mice administered by sniffing. In serum of mice administered by perfusion, although the IL-10 secretion was lower than that of mice administered by sniffing, a still significant level of IL-10 secretion was observed (Fig. 7) - In control mice infected with SeV-luc (7 x 10⁷ pfu/100 µl), there was no significant secretion of IL-10.

### Example 9: Intramuscular administration of Sendai virus vector into mice

SeV-βgal was injected into the TA muscles of mice, and the expression level of βgal after two days was examined by the β-galactosidase assay. The expression of βgal increased in a dose-dependent manner (Fig. 8). The expression level (61, 672 pg βgal/mg protein) obtained by the administration of SeV-βgal at the highest titer (3.5 x 10⁸ pfu) was 300 fold and 7 fold higher than those achieved with naked DNA and AAV (βgal AAV), respectively, at their optimized dose.

Time course of βgal expression after SeV-βgal administration was examined. SeV-βgal (3.5 x 10⁷ pfu/muscle) or control SeV-luc (3.5 x 10⁷ pfu/muscle) was injected into the TA muscle, and mice were sacrificed at different times, and the βgal expression in muscle homogenates was determined. The result showed that the expression of the transfected gene reached the maximal level two days after injection, and then declined to the basal level, the level before injection, after 28 days (Fig. 9).

The expression level obtained by repetitive administration of the Sendai virus vector was examined. Each of the TA muscles of mice was infected with SeV-βgal (3.5 x 10⁷ pfu/muscle), and further infected with SeV-luc (3.5 x 10⁷ pfu/muscle) at the indicated time. Mice were sacrificed after two days of the second injection, and luciferase expression in muscle homogenates were examined. The level of luciferase was compared with that of mice with a single injection of SeV-luc, or that of mice without a SeV-luc injection. By repetitive injection, the expression level was lowered, but still a significant increase in expression level was observed after the second injection (Fig. 10). A significant increase in the expression level was still obtainable by repetitive injections after 28 days of the initial injection (the expression level was decreased to 1/65 of that achieved by a single administration).

Increase of systemic anti-SeV antibodies induced by intramuscular administration of SeV was examined. Each of the TA muscles of mice was infected with SeV-βgal (3.5x10⁷ pfu/muscle), and mice were sacrificed at the indicated times, and SeV-specific antibodies in serum were detected. As shown in Fig. 11, dose-dependent antibody reaction was clearly observed systemically. In addition, inflammation in TA, which was also dependent on the SeV dose, was observed. The antibody reaction and inflammation in TA caused by SeV administration might be the reason for the decreased expression after repetitive injection.

### Example 10: Intramuscular administration of IL-10 expressing Sendai virus vector

SeV-IL10 was administered into TA muscle, and the IL-10 secretion efficiency was examined (4 x 10⁸ pfu/muscle). Two days after injection, muscle homogenates were prepared and IL-10 expression was determined. The result was compared with that obtained by plasmid DNA transfection mediated by liposome. In addition, IL-10 level in serum was also determined.

The level of IL-10 expression in muscle homogenates after two days of injection was found to be higher by one or two orders of magnitude than that obtained by plasmid DNA injection (50 µg DNA/TA) (SeV-IL10: 1067±32 pg/mg protein; plasmid: 50.9±11 pg/mg protein) (Fig. 12). The IL-10 level in serum was increased two days after SeV-IL10 administration, whereas IL-10 was not detected in the serum of mice that had been administered with plasmid DNA (Fig. 13).

In a separate experiment performed similarly to the above experiment, the IL-10 level in muscle homogenates after two days of SeV-IL10 injection was 160 fold higher than that obtained by transfection of naked plasmid DNA expressing IL-10 (Fig. 14). In contrast, in mice administered with IL-10 expressing AAV (IL-10 AAV), IL-10 was not detected in muscle homogenates two days after injection. IL-10 was not detected in sera of mice either transfected with plasmid DNA or infected with AAV, whereas the IL-10 level in the sera of mice injected with SeV-IL10 was increased to 797±383 pg/ml after two days of injection (Fig. 15). These data indicate that recombinant SeV can efficiently transfer genes through its intramuscular administration.

### Example 11: Effect of SeV-IL10 administration in the pulmonary fibrosis model mice

The effect of SeV-IL10 in model mice with bleornycin-induced pneumonitis and fibrosis was examined. SeV-IL10, or as controls, SeV-βgal and SeV-luc (each 7 x 10⁸ pfu/mouse) were administered to the TA muscle. Two days after intramuscular administration, bleomycin (0. 075 u/100 µl; 0.075 units contained in 100µl saline) or saline was injected intratracheally. Eleven days after bleomycin injection, mice were sacrificed, and the collagen deposition in lung was determined by a hydroxyproline assay according to the described method (Pettipher E.R., Br. J. Pharmacol., 1993, 110, 423-427) through determining the amount of 6-hydroxyproline.

Administration of SeV on its own was found to increase collagen deposition in the respiratory tract (Fig. 16; compare the 1st (SeV-βgal + SeV-luc + bleomycin) and 3rd (bleomycin) columns from the left). However, the administration of SeV-IL10 significantly reduced collagen deposition (compare the 1st (SeV-βgal + SeV-luc + bleomycin) and 2nd (SeV-IL10 + bleomycin) columns from the left). Thus, it is confirmed that intramuscular administration of SeV-IL10 has a therapeutic effect on pulmonary fibrosis.

### Industrial Applicability

The present invention enables a high level expression of a foreign gene in the cardiovascular system. It also provides a fundamental technology for gene therapy against a variety of inflammatory diseases including pulmonary fibrosis that is represented by cystic fibrosis.

## Claims

1. Use of a paramyxovirus vector comprising a foreign gene encoding a secretory protein for the preparation of a pharmaceutical composition for the treatment of an inflammatory disease, wherein said paramyxovirus vector is to be administered intramuscularly.

2. The use of claim 1, wherein said secretory protein is an anti-inflammatory cytokine.

3. The use of claim 2, wherein said anti-inflammatory cytokine is IL-10.

4. The use of any one of claims 1 to 3, wherein said paramyxovirus is Sendai virus.

5. The use of claim 1, wherein said inflammatory disease is pulmonary fibrosis.

## Patentansprüche

1. Verwendung eines Paramyxovirus-Vektors, umfassend ein Fremdgen, das ein sekretorisches Protein codiert, für die Herstellung eines Arzneimittels zur Behandlung einer Entzündungserkrankung, wobei der Paramyxovirus-Vektor intramuskulär zu verabreichen ist.

2. Verwendung nach Anspruch 1, wobei das sekretorische Protein ein antiinflammatorisches Cytokin ist.

3. Verwendung nach Anspruch 2, wobei das anti-inflammatorische Cytokin IL-10 ist.

4. Verwendung nach einem der Ansprüche 1 bis 3, wobei das Paramyxovirus ein Sendai-Virus ist.

5. Verwendung nach Anspruch 1, wobei die Entzündungserkrankung Lungenfibrose ist.

## Revendications

1. Utilisation d'un vecteur paramyxovirus comprenant un gène étranger codant une protéine de sécrétion pour la préparation d'une composition pharmaceutique pour le traitement d'une maladie inflammatoire, dans laquelle ledit vecteur paramyxovirus est à administrer par voie intramusculaire.

2. Utilisation selon la revendication 1, dans laquelle ladite protéine de sécrétion est une cytokine anti-inflammatoire.

3. Utilisation selon la revendication 2, dans laquelle ladite cytokine anti-inflammatoire est l'IL-10.

4. Utilisation selon l'une quelconque des revendications 1 à 3, dans laquelle ledit paramyxovirus est le virus de Sendai.

5. Utilisation selon la revendication 1, dans laquelle ladite maladie inflammatoire est la fibrose pulmonaire.


### SEQUENCE LISTING

<110> DNAVEC Research Inc.
<120> PARAMYXOVIRUS VECTOR FOR GENE TRANSFER TO THE CARDIOVASCULAR SYSTEM
<130> D3-A0008P
<140>
   <141>
<150> JP 2000-339942
   <151> 2000-11-08
<160> 8
<170> PatentIn Ver. 2.0
<210> 1
   <211> 10
   <212> DNA
   <213> Artificial Sequence
<220>
   <223> Description of Artificial Sequence: Artificially Synthesized Sequence
<400> 1
   ctttcaccct                                                          10
<210> 2
   <211> 15
   <212> DNA
   <213> Artificial Sequence
<220>
   <223> Description of Artificial Sequence: Artificially Synthesized Sequence
<400> 2
   tttttcttac tacgg 15
<210> 3
   <211> 18
   <212> DNA
   <213> Artificial Sequence
<220>
   <223> Description of Artificial Sequence: Artificially Synthesized Sequence
<400> 3
   cggccgcaga tcttcacg 18
<210> 4
   <211> 18
   <212> DNA
   <213> Artificial Sequence
<220>
   <223> Description of Artificial Sequence: Artificially Synthesized Sequence
<400> 4
   atgcatgccg gcagatga 18
<210> 5
   <211> 18
   <212> DNA
   <213> Artificial Sequence
<220>
   <223> Description of Artificial Sequence: Artificially Synthesized Primer Sequence
<400> 5
   gttgagtact gcaagagc 18
<210> 6
   <211> 42
   <212> DNA
   <213> Artificial Sequence
<220>
   <223> Description of Artificial Sequence: Artificially Synthesized Primer Sequence
<400> 6
   tttgccggca tgcatgtttc ccaaggggag agttttgcaa cc
   42
<210> 7
   <211> 18
   <212> DNA
   <213> Artificial Sequence
<220>
   <223> Description of Artificial Sequence: Artificially Synthesized Primer Sequence
<400> 7
   atgcatgccg gcagatga
<210> 8
   <211> 21
   <212> DNA
   <213> Artificial Sequence
<220>
   <223> Description of Artificial Sequence: Artificially Synthesized Primer Sequence
<400> 8
   tgggtgaatg agagaatcag c 21